# EUROPÄISCHE PATENTSCHRIFT

(11) **EP 2 710 115 B1**
(45) Veröffentlichungstag und Bekanntmachung des Hinweises auf die Patenterteilung: **27.12.2017**
(21) Anmeldenummer: 12726756.5
(22) Anmeldetag: 16.05.2012
(51) Int. Cl.: C12N 1/20, C12R 1/225, A61K 35/74, A23C 9/123

(54) **NEUE MILCHSÄUREBAKTERIEN UND DIESE ENTHALTENDE ZUSAMMENSETZUNGEN GEGEN BAKTERIELLE ERKÄLTUNGEN**
NOVEL LACTIC ACID BACTERIA AND COMPOSITIONS CONTAINING THEM AGAINST BACTERIAL COMMON COLDS
NOUVELLES BACTÉRIES D'ACIDE LACTIQUE ET COMPOSITION LES CONTENANT CONTRE DES INFECTIONS BACTÉRIENNE

(30) Priorität: 16.05.2011 EP 11166203
(43) Veröffentlichungstag der Anmeldung: 26.03.2014
(73) Patentinhaber: Belano Medical AG, 13355 Berlin (DE)
(72) Erfinder: LANG, Christine, 14057 Berlin (DE); RAAB, Andreas, 10589 Berlin (DE); BOLOTINA, Natalia, 10777 Berlin (DE)
(74) Vertreter: Hertin, Paul W.
(86) Internationale Anmeldenummer: PCT/EP2012/059213
(87) Internationale Veröffentlichungsnummer: WO 2012/156491

(56) Entgegenhaltungen:
- WO-A1-2007/073709
- KELLER METTE KIRSTINE ET AL: "Co-aggregation and growth inhibition of probiotic lactobacilli and clinical isolates of mutans streptococci: An in vitro study", ACTA ODONTOLOGICA SCANDINAVICA, OSLO, NO, Bd. 69, Nr. 5, 9. Februar 2011 (2011-02-09), Seiten 263-268, XP009162555, ISSN: 0001-6357
- TWETMAN LISA ET AL: "Coaggregation between probiotic bacteria and caries-associated strains: An in vitro study", ACTA ODONTOLOGICA SCANDINAVICA, OSLO, NO, Bd. 67, Nr. 5, 1. September 2009 (2009-09-01), Seiten 284-288, XP009138868, ISSN: 0001-6357, DOI: 10.1080/00016350902984237
- EVA M SÖDERLING ET AL: "Probiotic Lactobacilli Interfere with Biofilm Formation In Vitro", CURRENT MICROBIOLOGY, SPRINGER-VERLAG, NE, Bd. 62, Nr. 2, 11. September 2010 (2010-09-11), Seiten 618-622, XP019873919, ISSN: 1432-0991, DOI: 10.1007/S00284-010-9752-9
- LANG C ET AL: "Specific Lactobacillus/Mutans Streptococcus co-aggregation", JOURNAL OF DENTAL RESEARCH, INTERNATIONAL & AMERICAN ASSOCIATION FOR DENTAL RESEARCH, Bd. 89, Nr. 2, 1. Februar 2010 (2010-02-01), Seiten 175-179, XP009162445, ISSN: 1544-0591
- GUGLIELMETTI SIMONE ET AL: "Oral Bacteria as Potential Probiotics for the Pharyngeal Mucosa", APPLIED AND ENVIRONMENTAL MICROBIOLOGY, AMERICAN SOCIETY FOR MICROBIOLOGY, US, Bd. 76, Nr. 12, 1. Juni 2010 (2010-06-01), Seiten 3948-3958, XP009139501, ISSN: 0099-2240, DOI: 10.1128/AEM.00109-10 [gefunden am 2010-04-23]
- GUGLIELMETTI SIMONE ET AL: "A Dairy Bacterium Displays In Vitro Probiotic Properties for the Pharyngeal Mucosa by Antagonizing Group A Streptococci and Modulating the Immune Response", INFECTION AND IMMUNITY, AMERICAN SOCIETY FOR MACROBIOLOGY, Bd. 78, Nr. 11, 1. November 2010 (2010-11-01), Seiten 4734-4743, XP009162663, ISSN: 0019-9567
- L. MAUDSDOTTER ET AL: "Lactobacilli Reduce Cell Cytotoxicity Caused by Streptococcus pyogenes by Producing Lactic Acid That Degrades the Toxic Component Lipoteichoic Acid", ANTIMICROBIAL AGENTS AND CHEMOTHERAPY, Bd. 55, Nr. 4, 1. April 2011 (2011-04-01), Seiten 1622-1628, XP55037671, ISSN: 0066-4804, DOI: 10.1128/AAC.00770-10
- RICKARD ALEXANDER H ET AL: "Bacterial coaggregation: An integral process in the development of multi-species biofilms", TRENDS IN MICROBIOLOGY, ELSEVIER SCIENCE LTD., KIDLINGTON, GB, Bd. 11, Nr. 2, 1. Februar 2003 (2003-02-01), Seiten 94-100, XP009154671, ISSN: 0966-842X

## Beschreibung

Die vorliegende Erfindung betrifft einen Mikroorganismus, der Ordnung Milchsäurebakterien, wobei der Mikroorganismus mit *Streptococcus pyogenes* koaggregieren kann. Des Weiteren betrifft die Erfindung Zusammensetzungen, die die Milchsäurebakterien oder deren Zelllysat enthalten, insbesondere zur Verwendung in der Körperhygiene und Krankheitstherapie. Insbesondere betrifft die vorliegende Erfindung die Verwendung der neuen Milchsäurebakterien bzw. der dieser enthaltenden Zusammensetzungen zur Behandlung und/oder Prophylaxe aller Erkrankungen, welche durch *Streptococcus pyogenes* hervorgerufen werden können.

Des Weiteren stellt die hier beschriebene Entwicklung ein innovatives biologisches Produkt in Form von GRAS-Mikroorganismen bzw. Milchsäurebakterien dar, das als spezifisch wirkendes antimikrobielles Additiv für die Prophylaxe und die lokale Behandlung von Entzündungen im Mund- und Rachenraum sowie für Infektionen des oberen Respirationstraktes und der Haut eingesetzt werden kann.

Ferner betrifft die vorliegende Erfindung die Verwendung des erfindungsgemäßen Milchsäurebakteriums oder dessen Zelllysat in Zusammensetzungen oder pharmazeutischen Produkten oder Medizinprodukten (Mundhygiene), beispielsweise in Form von Sprays, Gurgel- oder Spüllösungen, als Lutsch- oder Halstabletten, Pastillen, Dragees, Aerosolen, Zahncremes, Säften, Sirup oder als Zusatz in Lebensmitteln bzw. als Nahrungsergänzungsmittel.

### HINTERGRUND

Mit einer Hals- oder Mandelentzündungen (Pharyngitis) sind die meisten Menschen bereits in Berührung gekommen. Die Krankheit kann sowohl virale als auch bakterielle Ursachen haben. Etwa 20 - 30 % der Hals- und Mandelentzündungen sind bakteriell bedingt. Hier liegt in jedem Fall eine Infektion durch *Streptococcus pyogenes* vor. *Streptococcus pyogenes* ist eines der häufigsten humanpathogenen Bakterien. Die Verbreitung der bakteriellen Pharyngitis erfolgt über Tröpfcheninfektion. Die am häufigsten von der Infektion betroffene Gruppe sind Kinder und Jugendliche im Alter zwischen 5 und 15 Jahren, in gedrängten Menschenansammlungen werden jedoch auch ältere Menschen infiziert (Bisno 1995). In den USA wird die Zahl der S. *pyogenes* Infektionen auf 10 Millionen pro Jahr geschätzt (Kilian 2002). Die damit verbundenen Kosten belaufen sich für die USA auf schätzungsweise 1 Milliarde $ jährlich (Reid et al. 2001). Die Zahl der akuten Streptokokken-Pharyngitiden in Deutschland wird auf 1 bis 1,5 Millionen pro Jahr geschätzt. Nur ein Teil der Infektionen verläuft klinisch apparent, d.h. das Reservoir der übertragenen Erreger ist größer.

*Streptococcus pyogenes* (Streptokokken der Gruppe A oder GAS genannt) ist ein humanpathogener Erreger und gehört zu den grampositiven Kokken, die sich in Ketten aneinanderreihen. Wie viele andere Krankheitserreger weisen auch Streptokokken die Fähigkeit zur Expression mehrerer Virulenzfaktoren auf. Der erste Schritt im Laufe der Pathogense von *Streptococcus pyogenes* ist die Adhäsion der Bakterien an die Oberfläche der Wirtszellen. Nach einem weitgehend akzeptierten Modell erfolgt die Adhäsion in zwei Stufen: die erste Stufe besteht aus einer schwachen, relativ unspezifischen Interaktion mit der Oberfläche von humanen Zellen, in deren Folge es unmittelbar zu einer gewebsspezifischen, hochaffinen Interaktion kommt (Hasty et al. 1992; Courtney et al. 1999; Cunningham 2000). Bereits 1976 konnten Beachey und Mitarbeiter die Lipoteichonsäure (LTA) als ein Molekül identifizieren, dass auf bakterieller Seite die initiale Adhäsion vermittelt. Auf Seite des Epithels konnte Fibronectin als Rezeptor für LTA ausgemacht werden (Simpson und Beachey 1983). Für die zweite Stufe der Adhäsion sind mindestens elf weitere Strukturen auf der bakteriellen Oberfläche identifiziert, die eine Bindung an die Epithelzellen vermitteln. Der zweite Schritt in der Pathogenese durch *Streptococcus pyogenes* ist die Invasion der Bakterien in die epithelenWirtszellen (LaPenta et al. 1994). LaPenta und Kollegen konnten zeigen, dass GAS humane Epithelzellen mit Frequenzen infizieren können, die teilweise über denen klassischer intrazellulärer humaner Pathogene wie z.B. *Salmonella* oder *Listeria* liegen.

Die pathogenen Bakterien innerhalb der Wirtszelle haben ein hohes Potential zur Vermehrung, was zu einer akuten Infektion führen kann. Das Überleben im Ruhezustand kann zum erneuten Auftreten von bakteriellen Infektionen der Schleimhäute und Epithelium führen, was die wiederkehrenden Infektionen der Mund-Rachen-Schleimhaut erklären kann. Insbesondere Angina ist eine wiederkehrende Infektion, die sehr häufig bei Kindern durch GAS verursacht wird.

*Streptococcus pyogenes* ist der ätiologische Agent mehrerer akuter Krankheiten, wie Pharyngitis, Scharlach, Impetigo, Cellulitis. Auch invasive, toxigene Infektionen, wie nekrotisierende Fasziitis, Myositis und Streptokokken-induziertes Toxisches Schock-Syndrom sowie die Entwicklung von immunvermittelten Folgekrankheiten, wie Rheumatisches Fieber und Glomerulonephritis, werden durch *Streptococcus pyogenes* verursacht.

Es wird geschätzt, dass zwischen 5 und 15 % aller Menschen das Bakterium (üblicherweise im Rachen) tragen, ohne Zeichen einer Erkrankung. Als Teil der normalen Flora kann *Streptococcus pyogenes* dann infizieren, wenn die Immunabwehr geschwächt ist. Der Kolonisation von Geweben (z.B. des Respirationstraktes oder der Haut) mit *Streptococcus pyogenes* folgt der Krankheitsausbruch verbunden mit den einschlägigen Symptomen, wie z.B. Kratzen im Rachen, Halsschmerzen und Schluckbeschwerden.

Ferner können Infektionen mit *Streptococcus pyogenes* zu Komplikationen durch Ausbreitung der Infektion in den tieferen Respirationstrakt (Otitis media, Sinusitis, Pneumonia) oder in die Blutbahn zu Meningitis sowie zu Infektionen der Knochen (Osteomyelitis) und der Gelenke (Arthritis) führen.

Ein Element in der Kolonisierung ist die bakterielle Adhäsion an eine Zell- bzw. Schleimhautoberfläche. Das Bakterium besitzt ein großes Repertoire an Adhäsinen, welche die Bindung an Zelloberflächen vermitteln (z.B. M-Protein, Fibronektin-Binding-Protein, LTA, Collagen-Binding-Protein). Die Interaktion bzw. Bindung an Zelloberflächen spielt eine initiale Rolle in der Besiedlung von Wirtszellen sowie der Pathogenese von *Streptococcus pyogenes.*

Demensprechend ist der frühzeitige Einsatz geeigneter therapeutischer Mittel zur Prophylaxe und zur Behandlung von wesentlicher Bedeutung, um die Gesamtkeimzahl von GAS zu reduzieren und der Bindung bzw. Invasion von *Streptococcus pyogenes* effizient vorzubeugen.

Im Stand der Technik sind therapeutische Mittel beschrieben, die in der Lage sind die durch *Streptococcus pyogene* im Hals- und Rachenbereich hervorgerufene Beschwerden zu lindern.

### STAND DER TECHNIK

Die Standardtherapie einer akuten Erkrankung ist die Gabe von Antibiotika. Unter anderem aufgrund von verstärkt auftretenden Penicillin-Resistenzen seit Mitte der 1980er Jahre ist die Krankheit in den Industrieländern in den letzten Jahrzehnten wieder auf dem Vormarsch (Kaplan 1991; Musser und Krause 1998). Es gibt keine Prophylaxe, da bisher kein funktionierender Impfstoff entwickelt wurde. Es liegt an der Diversität des hauptsächlich genutzten Antigens, des M-Proteins aus der bakteriellen Zellwand und an der immunologischen Kreuzreaktivität der weiteren Antigenkandidaten mit humanen Proteinen.

Es ist allgemein gezeigt, dass trotz Antibiotika-Behandlung (in der Regel Penicillin oder Erythromycin), welche massive Nebenwirkungen ausüben können, die Infektion bei den meisten Patienten rezidiviert.

Bei den derzeit auf dem Markt befindlichen Präparaten handelt es sich im Wesentlichen um Lutschpastillen, Tabletten, Gurgellösungen und Rachensprays, teilweise versetzt mit Lokalanästhetika (Lidocain, Benzocain). Diese lindern die Beschwerden im Hals- und Rachenbereich nur kurzzeitig, eine Abtötung von *Streptococcus pyogenes* wird durch diese Präparate nicht erreicht.

Milchsäurebakterien werden im Allgemeinen als probiotische Bakterien für den Schutz vor Magen-Darm-Erkrankungen eingesetzt, die durch Krankheitserreger ausgelöst werden, da sie neben Milchsäure auch häufig anti-bakterielle Substanzen produzieren. Lactobazillen (Milchsäurebakterien) sind Gram-positive, anaerobe bis aerotolerante Bakterien, die in der Lage sind Zucker zu Milchsäure zu verstoffwechseln (Milchsäuregärung). Ihnen zugeordnet sind die Familien Lactobacillaceae, Aerococcaceae, Carnobacteriaceae, Enterococcaceae, Leuconostocaceae und Streptococcaceae. Sie gelten als apathogen und werden im Allgemeinen als probiotische Bakterien zur Verbesserung der Magen-Darm Flora sowie bei der Behandlung von gastrointestinalen Beschwerden eingesetzt. Laktobazillen sind insbesondere für die Nahrungsmittelindustrie von Bedeutung, wo sie im Bereich Functional Food' eine wesentliche Rolle spielen. Früher wurde die Art Bifidobacterium bifidum zu den Laktobazillen gestellt (*Lactobacillus bifidum*), wobei es nach heutigem Stand phylogenetisch mit der Ordnung nicht in näherer Verbindung steht. Den Stoffwechsel betreffend wird es aber weiterhin als Milchsäurebakterium behandelt. Milchsäurebakterien sind außerdem für die Lebensmittelindustrie von hoher Wichtigkeit, da sie für die Herstellung von Milchprodukten genutzt werden, aber auch als Schädlinge (z. B. in der Bierbrauerei) auftreten können. Die Milchsäurebakterien werden als apathogen bezeichnet.

Im Stand der Technik ist die Verwendung von probiotischen Bakterien für unterschiedliche Anwendungen beschrieben. So offenbart z. B. die WO 2010/130563 die Nutzung von probiotischen Bakterien für Geschirrspülmittel, wodurch die negativen Folgen des Handgeschirrspülens für die Haut verringert werden. Außerdem stellt sich ein pflegender Effekt für die Haut ein.

Der Einsatz derartiger Mikroorganismen in kosmetischen Hautbehandlungsmitteln ist bereits bekannt. So wird etwa in US 6790434 die Verwendung solcher Mikroorganismen in kosmetischen Hautbehandlungsmitteln in Verbindung mit einem pflanzlichen Extrakt aus der extrazellulären Matrix beschrieben, um hierdurch der durch UV-Strahlung bedingten Hautschädigung entgegen zu wirken. Jedoch wird der Einsatz dieser Mikroorganismen in Wasch- und Reinigungsmitteln nicht offenbart.

Weiterhin ist der Einsatz bestimmter Bacillus-Arten in Sanitärreinigungsmitteln bekannt. So wird in WO 97/25865 der Einsatz von Bacillus-Arten in Sanitärreinigungsmitteln beschrieben, die die Vermehrung von Pathogenen vermeiden und organischen Schmutz abbauen sollen.

Die offenbarten Verwendungen beinhalten oftmals inaktivierte Bakterien in Form von Sporen. Die Sporen werden durch Substrate reaktiviert und sollen durch genetische Defekte keine Vermehrung zeigen. Nachteilig hierbei ist jedoch, dass die Vermehrung bzw. Mutation von Bakterien nicht völlig ausgeschlossen werden kann, so dass eine Restgefahr besteht, dass die Bakterien sich unkontrolliert vermehren.

Eine orale Darreichungsform von probiotischen Bakterien ist mitunter in der WO 2005/117921 offenbart. Hierbei weist die Darreichungsform wenigstens eine Gattung von probiotischen Mikroorganismen auf, wobei die Darreichungsform und/oder die Bakterien mit einem Celluloseether enthaltenen Überzug versehen ist.

In *in vitro* Versuchen konnte gezeigt werden, dass einige *Lactobacillus* Stämme dazu in der Lage sind, die Bindung von GAS an menschliche Zelllinien zu verhindern. Die *Lactobacillus* Stämme konkurrieren mit GAS um Oberflächenstrukturen auf den Zellen und Verhindern somit die Invasion von GAS in die Wirtszellen. Da die Adhäsion von *Streptococcus pyogenes* an die Wirtszelle bereits der erste Schritt der Pathogenese dargestellt, und der Invasionsprozess in die Wirtszellen sehr kurzfristig erfolgt, ist dieses Verfahren als nachteilhaft bei der Behandlung von GAS Infektionen anzusehen. Wünschenswert wäre hier bereits eine Co-Aggregation von planktonischen *Streptococcus pyogenes* Zellen, bevor eine Bindung an die Zelloberfläche stattfindet und eine Immunantwort induziert werden kann.

Aufgabe der Erfindung ist, ein Mittel oder Zusammensetzung zur Prophylaxe und Behandlung von Infektionen hervorgerufen durch *Streptococcus pyogenes* bereitzustellen, das nicht die Mängel oder Nachteile des Standes der Technik aufweist.

### DARSTELLUNG DER ERFINDUNG

Gelöst wird die Aufgabe durch die unabhängigen Ansprüche. Vorteilhafte Ausführungen ergeben sich aus den Unteransprüchen.

In einem ersten Aspekt betrifft die Erfindung ein Milchsäurebakterium oder dessen Zelllysat zur Prophylaxe und/oder Therapie mikrobiell bedingter Erkrankungen der Haut, Schleimhäute und/oder der Mundhöhle,, wobei das Milchsäurebakterium, mit mindestens einem pathogenen Mikroorganismus koaggregieren kann, wobei der pathogene Mikroorganismus *Streptococcus pyogenes* ist und das Milchsäurebakterium ausgewählt ist aus der Gruppe umfassend folgende bei der Deutschen Sammlung für Mikroorganismen und Zellkulturen hinterlegten als DSM 25972, DSM 25987, DSM 25988, DSM 25989 und DSM 25973 nummerierten Mikroorganismen..

Es war völlig überraschend, dass ein Mikroorganismus bereitgestellt werden konnte, der an *Streptococcus pyogenes* bindet, wobei es sich bei dem Mikroorganismus um ein Milchsäurebakterium handelt. Zudem war es völlig überraschend, dass bei dem Milchsäurebakterium oder dessen Zelllysat in einer bevorzugten Ausführungsform die Fähigkeit zur Koaggregation des pathogenen Mikroorganismus auch nach einer biologischen, chemischen oder physikalischen Behandlung besteht. Vorteilhafterweise besteht die Fähigkeit zur Koaggregation des pathogenen Mikroorganismus auch bei einem pH-Wert von zwischen ca. 3 bis ca. 8. Es ist bevorzugt, dass das Milchsäurebakterium davon die Fähigkeit zur Hemmung einer Bildung eines Biofilms des pathogenen Mikroorganismus besitzt. Ferner ist bevorzugt, dass das Milchsäurebakterium oder dessen Zelllysat die Fähigkeit zur Verhinderung der Bindung an Fibronektin durch *Streptococcus pyogenes* aufweist. In einer bevorzugten Ausführungsform weist das Milchsäurebakterium oder dessen Zelllysat die Fähigkeit auf, keinen kommensalen Mikroorganismus der Haut oder der Schleimhäute zu koaggregieren.

Vorteilhafterweise ist das Milchsäurebakterium ausgewählt aus der Gruppe umfassend folgende bei der Deutschen Sammlung für Mikroorganismen und Zellkulturen hinterlegten als DSM 25972, DSM 25987, DSM 25988, DSM 25989, DSM 25973 nummerierten Mikroorganismen.

In einem weiteren Aspekt betrifft die Erfindung eine Zusammensetzung umfassend mindestens das Milchsäurebakterium oder dessen Zelllysat. Die Zusammensetzung kann bevorzugt einen pharmazeutisch oder kosmetisch verträglichen Träger oder Exzipienten umfassen. Es ist bevorzugt, dass die Zusammensetzung in fester, flüssiger, viskoser Form oder als Aerosol vorliegt. Insbesondere liegt die Zusammensetzung bevorzugt als Paste, Weichgelatinekapsel, Hartgelatinekapsel, Pulver, Granulat, Kügelchen, Pastille, Brausetablette, Lutschtablette, Buccaltablette, Kautablette, Subligualtablette, Lösung, Tinktur, Emulsion, Saft, Konzentrat, Sirup, Spray, Trinkampulle, Gel, Mundwasser, Zahnpulver, Kaugummi, Tablette, Dragee oder Bonbon vor.

Die Zusammensetzung kann vorteilhafterweise Probiotika, Antiseptika oder andere antibakteriell wirksame Substanzen umfassen und/oder bevorzugt zusätzlich enthaltend eine oder mehrere der nachfolgenden Substanzen, die aus Antioxidantien, Vitaminen, Coenzymen, Fettsäuren, Aminosäuren und Co-Faktoren ausgewählt sind .

In einer bevorzugten Ausführungsform der Erfindung handelt es sich bei der Zusammensetzung um eine pharmazeutische, tiermedizinische, kosmetische, Nahrungsergänzungsmittel oder Nahrungsmittelzusammensetzung. Die Zusammensetzung enthält bevorzugt ein oder mehrere Verdickungsmittel, ein oder mehrere Süßungsmittel und/oder ein oder mehrere künstliche Süßstoffe, wobei das Verdickungsmittel bevorzugt aus Celluloseether, Polysaccharide, ausgewählt aus der Gruppe umfassend Xanthangummi, Gelatine, hochdisperses Siliciumdioxid, Stärke, Alginate, Tragant, Agar-Agar, Gummi arabicum, Pektin und Polyvinylester ausgewählt ist und das Süßungsmittel ausgewählt ist aus der Gruppe umfassend Glucose, Fructose, Saccharose und Glucosesirup, Sorbitol, Mannitol, Xylitol und Maltitol, Saccharin, Natriumcyclamat, Acesulfam-K und/oder Aspartam.

Bevorzugte pharmazeutische Produkte im Sinne der Erfindung umfassen Nasenspülungen; Mund- und Zahnspülungen; Gurgellösungen, Nasensprays, Mundsprays, Rachensprays, Nasentropfen, Tropfen, Tinkturen, Saft oder Sirup gegen Husten, Halsschmerzen, Erkältungen oder Infekte im Mund, Hals oder Rachenbereich, Halstabletten, Bonbons, Kaubonbons, Dragees und Pastillen gegen Hals- oder Rachenbeschwerden oder Halsschmerzen sowie Aerosole.

Bevorzugte Medizinprodukte im Sinne der Erfindung umfassen Nasenspülungen; Mund- und Zahnspülungen; Gurgellösungen, Nasensprays, Mundsprays, Rachensprays, Nasentropfen, Tropfen, Tinkturen, Saft oder Sirup gegen Husten, Halsschmerzen, Erkältungen oder Infekte im Mund, Hals oder Rachenbereich, Halstabletten, Bonbons, Kaubonbons, Dragees und Pastillen gegen Hals- oder Rachenbeschwerden oder Halsschmerzen sowie Aerosole.

Bevorzugte kosmetische Produkte im Sinne der Erfindung umfassen Zahncreme, Lotion, Schüttelmixtur, Puder, Nasenspülungen, Mund- und Zahnspülungen, Gurgellösungen, Nasensprays, Mundsprays, Rachensprays, Kaugummi, Hydrogel, Creme, Cresa, Salbe, Fettsalbe oder Paste zum Aufbringen auf eine Hautfläche.

Bevorzugte Nahrungsmittel und Nahrungsergänzungsmittel im Sinne der Erfindung umfassen Brausetabletten, Vitamintabletten, Mineraltabletten, Spurenelementtabletten, Getränkepulver, Getränke, Säfte, Milchgetränke, Yoghurts, Mineralwasser, stille Wasser, Bonbons, Kaubonbons, Kaugummi, Saft oder Sirup, Halstabletten, Dragees und Pastillen sowie Aerosole. Zudem kann die Zusammensetzung weiterhin Buildersubstanzen, Enzyme, Elektrolyte, pH-Regulatoren, Verdicker, Soil release-Wirkstoffe, optische Aufheller, Vergrauungsinhibitoren, Farbübertragungsinhibitoren, Schaumregulatoren und/oder Farbstoffe enthalten.

Das Milchsäurebakterium kann vorteilhafterweise lebend oder in abgetöteter Form in der Zusammensetzung vorliegen. Weiterhin kann es bevorzugt sein, dass das Milchsäurebakterium in verkapselter, sprühgetrockneter und/oder lyophilisierter Form vorliegt, wobei es auch bevorzugt sein kann, dass das Milchsäurebakterium in Form eines Zelllysates vorliegt. Weiterhin ist bevorzugt, dass das Milchsäurebakterium in einer Menge mit einem Masseanteil von 0,001 Gewichtsprozent bis 20 Gewichtsprozent, bevorzugt bis 0,005 Gewichtsprozent bis 10 Gewichtsprozent, besonders bevorzugt 0,01 Gewichtsprozent bis 5 Gewichtsprozent in der Zusammensetzung vorliegt.

In einem weiteren Aspekt betrifft die Erfindung ein Verfahren zur Identifizierung und/oder Selektion eines Milchsäurebakteriums mit der Eigenschaft, *Streptococcus pyogenes* zu koaggregieren, wobei das Verfahren zumindest die folgenden Schritte aufweist:
a. Inkubieren des pathogenen Mikroorganismus zur Ausbildung eines Biofilmes,
b. Zugabe eines zu untersuchenden Milchsäurebakteriums und Inkubation zu einer Mischung zur Ausbildung der Koaggregation zwischen dem pathogenen Mikroorganismus und dem zu untersuchenden Milchsäurebakteriums,
c. Abtrennen der nicht gebundenen Milchsäurebakterien durch Entfernen des Überstandes und
d. Bestimmung des Biofilmes im Hinblick auf koaggregierte Milchsäurebakterien.

Außerdem kann das Verfahren zusätzlich den folgenden Verfahrensschritt umfassen:
- Untersuchung der Biofilm-Hemmung durch die pathogenen Mikroorganismen, wobei die Zugabe der zu untersuchenden Milchsäurebakterien während der Inkubation der Biofilm-bildenden pathogenen Mikroorganismen erfolgt und/oder bevorzugt
- Quantifizierung der Biofilmbildung nach Entfernung der nicht gebundenen Zellen mittels einer Messung der optischen Dichte im Vergleich zu einer Kontrolle ohne Zugabe der zu testenden Milchsäurebakterien.

Weiterhin betrifft die Erfindung die Verwendung der Zusammensetzung zur Herstellung eines Arzneimittels, Medizinproduktes oder Kosmetikums zur Behandlung, Prophylaxe und/oder Therapie mikrobiell bedingter Erkrankungen der Haut, der Schleimhäute und der Mundhöhle.

Die bevorzugte Zusammensetzung kann ferner zur topischen Prophylaxe und/oder Therapie mikrobiell bedingter Erkrankungen oder entzündlicher Erkrankungen der Haut, der Schleimhäute und der Mundhöhle verwendet werden. Es ist bevorzugt, dass umfasst die Zusammensetzung zur Prophylaxe und/oder Therapie mikrobiell bedingter Mundhöhlenerkrankungen verwendet wirdund zusätzlich ein oder mehrere Geschmacksstoffe umfasst. Weiterhin ist bevorzugt, dass die Zusammensetzung zur topischen Prophylaxe und/oder Therapie mikrobiell bedingter Erkrankungen oder entzündlicher Erkrankungen, vorzugsweise mikrobiell bedingter Erkrankungen oder entzündlicher Erkrankungen der Mundhöhle, in Form einer Kaumasse, eines Kaugummis, eines Bonbons, einer Pastille, einer Zahnpasta oder einer Mundspüllösung verwendet wird.

Es kann vorteilhaft sein, dass eine oder mehrere entzündungshemmende und/oder antimikrobielle Substanzen in der Zusammensetzung verwendet wird. Weiterhin kann es bevorzugt sein, dass die Zusammensetzung in Kombination mit Lösungsmitteln, Trägerstoffen, Hilfsstoffen, Füllstoffen, Geschmacksstoffen, Geruchsstoffen und/oder weiteren Inhaltsstoffen verwendet wird. In einer bevorzugten Ausführungsform kann die Zusammensetzung zur Herstellung eines Reinigungsmittels oder Desinfektionsmittels für die Behandlung von Oberflächen verwendet werden. Es ist zudem bevorzugt, dass die Zusammensetzung zur Herstellung eines Produktes, das im Bereich der Medizinprodukte und/oder Prophylaxe eingesetzt wird verwendet wird. Bevorzugt ist, wenn die Zusammensetzung zur Herstellung eines Mittels zur oralen, sublingualen, buccalen Aufnahme verwendet wird.

Die Zusammensetzung kann in einer bevorzugten Ausführungsform zur Herstellung eines spezifisch wirkendes antimikrobielles Additiv für die lokale Behandlung von Entzündungen im Mund- und Rachenraum sowie für Infektionen des oberen Respirationstraktes und der Haut verwendet werden. Die Verwendung einer bevorzugten Zusammensetzung erfolgt insbesondere prophylaktisch oder kurativ. Es kann bevorzugt sein, wenn die Zusammensetzung oral, sublingual, buccal appliziert wird.

Ferner betrifft die Erfindung ein Kit zur Hygienebehandlung, umfassend bevorzugte Milchsäurebakterien oder eine bevorzugte Zusammensetzung körperhygienische Vorrichtungen oder Geräte, Spülungen und/oder Pasten. Das Kit kann zur Behandlung von bakteriellen Infektionen im Mund- oder Rachenbereich eingesetzt werden, wobei die Infektionen insbesondere durch *Streptococcus pyogenes* verursacht sind, oder dieser zumindest an der Infektion beteiligt ist.

Die vorliegende Erfindung betrifft bevorzugt neue Milchsäurebakterien sowie Zusammensetzungen, die diese enthalten, insbesondere zur Verwendung zur Behandlung oder Prophylaxe von Säuglingen, Kleinkindern, Kindern, gesunden Menschen, alten Menschen, immunsupprimierten Menschen, Menschen mit einmaligen oder rezidivierenden *Streptococcus pyoge*nes Infekten bzw. Menschen mit bakteriell verursachten Hals- und Mandelentzündungen (Pharyngitis). Überraschenderweise kann die Erfindung auch für Tiere verwendet werden.

Dementsprechend ist es bevorzugt, die Zusammensetzung zur Herstellung eines Arzneimittels zu verwenden, das zur Behandlung oder Prophylaxe von Hals- und Rachenbeschwerden, Halsschmerzen, Rötungen des Halses oder des Rachens sowie eitrigen und nichteitrigen Hals- und Mandelentzündungen, bei denen die Gabe von Milchsäurebakterien wünschenswert ist, nützlich ist. Die bevorzugte Zusammensetzung kann kurativ oder prophylaktisch, z.B. als Probiotikum, verwendet werden.

Die bevorzugten Milchsäurebakterien haben insbesondere die Fähigkeit zur Koaggregation, mit spezifischer Bindung an *Streptococcus pyogenes.* Es war völlig überraschend, dass die bevorzugten Milchsäurebakterien keine Koaggregation oder Bindung der kommensalen Mikroorganismen, wie *Streptococcus salivarius,* bewirken. *Streptococcus salivarius* besiedelt die Schleimhäute im Hals- und Rachenbereich von gesunden Menschen und trägt zum gesunden mikrobiellen Gleichgewicht bei, daher ist dessen Aggregation im Rahmen der vorliegenden Erfindung nicht bevorzugt.

Das heißt, die bevorzugten Milchsäurebakterien koaggregieren spezifisch mit den pathogenen Bakterien *Streptococcus pyogenes.* Nichts dergleichen geht aus dem Stand der Technik hervor. Im Stand der Technik sind lediglich Milchsäurebakterien beschrieben, die die Besiedlung und Invasion von *Streptococcus pyogenes* an bzw. in humane Zelllinien verhindern. Die Adhäsion stellt aber bereits den essentiellen Schritt der bakteriellen Pathogenese dar, so dass dieser Ansatz nicht ohne Nebenwirkungen hervorzurufen, eingesetzt werden könnte. Im Gegensatz zeigen die bevorzugten Milchsäurebakterien eine spezifische Koaggregation mit planktonischen *Streptococcus pyogenes* Zellen, exemplarisch dargestellt an Stämmen mit den DSM Nummern ATCC 12344 und ATCC 19615 bevor diese an die Wirtszellen binden können. Somit ist die Erfindung als Abkehr vom Üblichen anzusehen, da im Stand der Technik der Trend besteht, die Bindung von *Streptococcus pyogenes* an die Epithelzellen zu reduzieren. Es ist jedoch nicht beschrieben, dass die pathogenen Zellen bereits in der Mundhöhle koaggregiert werden können, so dass eine Bindung an Epithelzellen von Vorneherein nicht erfolgen kann. Dies hat zudem den wesentlichen Vorteil, dass die Zellaggregate mit dem Speichel oder mittels einer Spüllösung herunter geschluckt werden und die *Streptococcus pyogenes* Zellen während der Magen-Darm Passage abgetötet werden. Es handelt sich somit auch um eine anwenderfreundliche Benutzung, da es generell als aufwendig und auch unangenehm empfunden wird, z. B. Spüllösungen wieder auszuspucken. Nichts dergleichen ist für die bevorzugte Verwendung nötig.

Dem Fachmann ist bekannt, dass die gesunde Haut bzw. Schleimhaut mit Mikroorganismen, wie Bakterien und Pilzen, die als Kommensalen bzw. Mutualen bezeichnet werden, dicht besiedelt ist. Die Mikroorganismen stellen einen natürlichen Bestandteil der Hautoberfläche dar und werden insbesondere als Hautflora zusammengefasst. Die Mikroorganismen, die unter dem Begriff der autochthonen Flora zu subsumieren sind, stellen eine wichtige Voraussetzung dar, um die Haut selbst und den Organismus als Ganzen vor pathogenen Keimen zu schützen und sind Teil des Mikrobioms. Diesbezüglich ist es besonders vorteilhaft, dass die bevorzugten Milchsäurebakterien die Hautflora nicht aus dem Gleichgewicht bringen, sondern lediglich an das pathogene Bakterium *Streptococcus pyogenes* binden bzw. diesen koaggregieren.

Nicht zuletzt zeigen die erfindungsgemäßen Milchsäurebakterien eine Verhinderung der Bindung an Fibronectin und damit eine Verhinderung der Zellinvasion. Dadurch, dass die bevorzugten Milchsäurebakterien *Streptococcus pyogens* koaggregieren, bzw. adhäsive Eigenschaften gegenüber diesen zeigen, führt dieses insbesondere zu einer Maskierung und somit zu einer Verdeckung von Pathogenitätsfaktoren, was zu einer Reduktion der bakteriellen Belastung und/oder zur Inhibierung der Bindung an Fibronectin bzw. an Hals- und Rachenzellen führt.

Auch wenn die Erfindung insbesondere eine Gruppe Milchsäurebakterien betrifft, ist die Einheitlichkeit der anmeldungsgemäßen Lehre gegeben. Die beanspruchten Milchsäurebakterien haben eine gemeinsame Eigenschaft oder Wirkung. Die Summe der strukturellen oder funktionellen Gemeinsamkeiten führt zu dem funktionellen Zusammenhang der Koaggregation von *Streptococcus pyogenes,* der Nicht-Bindung kommensaler Mikroorganismen der Haut bzw. Schleimhaut und der Verhinderung der Bindung an Fibronectin durch *Streptococcus pyogenes.* Die gemeinsamen Merkmale stellen daher keine willkürliche Summe von Merkmalen dar, sondern sind sozusagen der gemeinsame Fingerprint der beanspruchten Milchsäurebakterien, der vorteilhafterweise die Tauglichkeit der Milchsäurebakterien für den Zweck ermöglicht und charakterisiert. Außerdem hat sich überraschenderweise herausgestellt, dass die überraschende Eigenschaft der erfindungsgemäßen Milchsäurebakterien, nämlich die Koaggregation von *Streptococcus pyogenes* weder im Speichel noch in Gegenwart von Zuckern gehemmt wird. Dies ist insbesondere deswegen von Vorteil, da der bevorzugte Einsatzort der Milchsäurebakterien und der Zusammensetzung im Mundraum ist und aus dem Stand der Technik bekannt ist, dass Koaggregationsvorgänge im Allgemeinen durch hohe Zuckerkonzentrationen gehemmt werden. Versuche haben jedoch gezeigt, dass dies nicht für die erfindungsgemäßen Milchsäurebakterien gilt, da diese auch bei hohen Zuckerkonzentrationen (wie sie z. B. im Speichel vorliegen sowie in möglichen Applikationsformen, wie Brausetabletten, Vitamintabletten, Mineraltabletten, Spurenelementtabletten, Getränkepulver, Getränke, Säfte, Milchgetränke, Yoghurts, Bonbons, Kaubonbons, Kaugummi, Saft oder Sirup, Halstabletten, Dragees und Aerosole) *Streptococcus pyogenes* koaggregieren.

Es war zudem überraschend, dass die bevorzugten Milchsäurebakterien auch auf der Haut eingesetzt werden können und hier ebenfalls *Streptococcus pyogenes* koaggregieren. Dem Fachmann ist bekannt, dass *Streptococcus pyogenes* auch als pathogener Wundkeim bekannt ist. Somit ist es besonders vorteilhaft, dass die Koaggregationsfähigkeit der bevorzugten Milchsäurebakterien nicht auf den Mundraum beschränkt ist, sondern auch auf Hautpartien angewendet werden kann.

Die vorliegende Erfindung betrifft somit auch Milchsäurebakterien oder deren Zelllysate, sowie Zusammensetzungen, die diese enthalten, insbesondere zur Verwendung zur Behandlung oder Prophylaxe von Säuglingen, Kleinkindern, Kindern, gesunden Menschen, alten Menschen, immunsuppremierten Menschen, Menschen mit krankhaften Hautveränderungen (insbesondere Staphylococcal scalded skin syndrome, Impetigo contagiosa, Folliculitis superficialis, Impetiginisation, Hautabszesse, Furunkel (Furunkulose), Karbunkel (Eiterbeule), Phlegmone, trockene Haut, juckende Haut, gerötete Haut, irritierte Haut, stark fettende Haut, Akne, diabetischer Fuß, Dekubitus, Neurodermitis, Akute Lymphadenitis, Pilonidalzysten (inkl. Pilonidalfistel, Pilonidalsinus, Steißbeinfistel, Steißbeinzyste), sonstige lokale Infektionen der Haut und der Unterhaut (z.B. Pyodermie, Dermatitis purulenta, Dermatitis septica, Dermatitis suppurativa); des Weiteren Dermatitis und Ekzeme (z.B. atopisches Ekzem, seborrhoisches Ekzem, Windeldermatitis, allergische Kontaktdermatitis, seborrhoisches Dermatitis, exfoliative Dermatitis, toxische Kontaktdermatitis, Lichen simplex chronicus, Prurigo, Pruritus sowie sonstige Dermatitis; weiterhin papulosquamöse Hautkrankheiten (Psoriasis, Parapsoriasis), Krankheiten der Hautanhangsgebilde (z.B. narbige Alopezie inkl. Folliculitis decalvans), sowie sonstige Erkrankungen der Haut und Unterhaut (z.B. Ulcus cruris), Personen mit bestehenden Schäden an der Haut (z.B. trockene Haut), Hautverletzungen (z.B. Schürfwunden, Wunden, auch nach Unfällen oder Operationen) oder von Nutz- und Haustieren eingesetzt werden kann.

Im Sinne der Erfindung wird die Haut in bevorzugten Ausführungsformen insbesondere als äußeres Organ des menschlichen oder tierischen Organismus verstanden, welches der Abgrenzung von innen und außen dient. Zu einem Hautareal im Sinne der Erfindung gehören in bevorzugten Ausführungsformen Bestandteile der Oberhaut, der Lederhaut oder der Unterhaut. Die Oberhaut (Epidermis) besteht erfindungsgemäß aus folgenden Schichten: Hornschicht (Stratum corneum), Leuchtschicht (Stratum lucidum), Körnerschicht (Stratum granulosum), Stachelzellschicht (Stratum spinosum) und/oder Basalschicht (Stratum basale). Jede Modifikation von Zellen in diesem Bereich ist eine Zellmodifikation in einem Hautareal im Sinne der Erfindung. Die Lederhaut (Dermis, Corium), die ebenfalls ein Bestandteil der erfindungsgemäßen Hautareale sein kann, besteht vorwiegend aus Bindegewebsfasern und dient der Ernährung und Verankerung der Epidermis. Das kapillarisierte Blutgefäßsystem in der Grenzzone zur Epidermis gehört mit zum Hautareal im Sinne der Erfindung ebenso wie die Talg- und Schweißdrüsen. Die Dermis im Sinne der Erfindung kann in ein Stratum papillare und in ein Stratum reticulare unterteilt werden. Weiterhin kann ein Hautareal im Sinne der Erfindung jedes Areal, d. h. auch jeder Ort in oder an der Unterhaut (Subcutis) oder eines Gewebes im Inneren des Körpers oder einer jeden Organes oder Organbestandteiles sein. Eine Gewebebarriere, die ein Organ von den umgebenden Strukturen abgrenzt, kann eine Haut im Sinne der Erfindung sein. Weiterhin werden durch den erfindungsgemäßen Begriff des Hautareals auch Hautanhanggebilde umfasst, wie Haare, Talgdrüsen, Haarbalgmuskeln, Nägeln, Hörner und Schweißdrüsen, insbesondere die ekkrinen und die apokrinen Schweißdrüsen, aber auch die Milchdrüsen. Jede Zellmodifikation, insbesondere ein vom normalen abweichendes Zellwachstum, kann mit den erfindungsgemäßen Mitteln behandelt werden, bevorzugt ohne auf die äußeren Hautareale beschränkt zu sein. Die Hautareale im Sinne der Erfindung können aber auch die Leistenhaut betreffen, wie sie an den Fingern oder an der Fußsohle auftreten oder aber die Felderhaut und die hiermit assoziierten Hautanhangsgebilde.

Die bevorzugte Zusammensetzung kann insbesondere in einer Seife, einer Lotion, einem Puder, einem Syndet, einem Schaum, einem Stift, einer Emulsion, einem Spray, einem Creme, einem Gel, einem Shampoo, einer Flüssigseife oder einem Deo enthalten sein. Ferner ist es bevorzugt, die Zusammensetzung, insbesondere als Probiotikum zu verwenden, das als Wasch-, Spül-, Reinigungs-, oder Desinfektionsmitteln (z.B. Seifen, Pulvern, Pasten, Lösungen, Emulsionen, Lotionen), Reinigungs- und/oder Desinfektionstüchern, Shampoos, Spülungen oder Anwendungen für Haut, Haare und/oder Kopfhaut, Cremes, Salben, Hautreinigungs- und/oder -pflegelotionen, in Lösungen (z.B. als Tropfen, Spray, Spülung) zur Anwendung in oder an Augen, Ohren, Mund, Nasen-, Rachenraum zugesetzt und/oder in Bandagen oder Wundauflagen eingebracht werden kann, um die Bildung von pathogenen Leitkeimen zu unterbinden, diese zu binden, diese als Aggregat zu entfernen und/oder sie zu hemmen bzw. abzutöten und sie so zu reduzieren.

Es war völlig überraschend, dass die Vorteile der erfindungsgemäßen Zusammensetzung noch einmal verbessert werden können, indem es in die genannten galenischen Formen eingebracht wird. Dem Fachmann sind weitere Formulierungskonzepte für das Einbringen der erfindungsgemäßen Zusammensetzung z. B. in Trägersubstanzen bekannt wie z. B. Emulsionen, oder andere Produkte zur dermalen Applikation wie z. B. flüssige Formen, die bevorzugt wasserfrei oder wasserhaltig sein können, wobei die wasserhaltigen erfindungsgemäß in Einphasensysteme und in Mehrphasensysteme unterschieden werden können. Weiterhin können halbfeste Formen, die wasserfrei oder wasserhaltig sind, eingesetzt werden, wobei wiederum eine Einteilung in Einphasensysteme und Mehrphasensysteme bei den wasserhaltigen halbfesten Formen möglich ist. Weiterhin können bevorzugt feste Formen eingesetzt werden, die lipophil oder hydrophil sind. Beispiele für solche Formen sind neben den genannten z. B. Fettsalben, Schäume, Puder, Stifte, Gelcremen, Hydrodispersionsgele, dünnflüssige Emulsionen, Lotionen, Salben, Sprays und Cremes. Hierbei ist dem Fachmann bekannt, dass sich derartige Trägersubstanzen zum einen vom Hautgefühl her in reichhaltig/wertvolle und frische und leichte unterscheiden lassen und zum anderen im Hinblick auf die Viskosität in niedrigviskose und in hochviskose. Nanoemulsionen oder Öle bzw. Oleogele weisen eher eine niedrige Viskosität auf, wohingegen Hydrogele oder Hydrocremen bzw. O/W-Emulsionen oder W/O-Emulsionen eine hohe Viskosität aufweisen. Wenn flüssige Applikationsformen zum Einsatz kommen, können diese - wie oben ausgeführt - in wasserfreie und wasserhaltige Systeme unterteilt werden. Bei den wasserfreien Systemen sind insbesondere apolare Systeme, polare Systeme ohne Emulgatoren und polare Systeme mit Emulgatoren bevorzugt. Bei den wasserhaltigen Systeme sind einphasige Systeme wie Lösungen und Mikroemulsionen bevorzugt, bei den mehrphasigen Systemen sind multiple Emulsionen, W/O-Emulsionen oder O/W-Emulsionen bevorzugt. Bei den Fest/Flüssig-Systemen sind bevorzugte Formen die Suspensionen oder die Flüssig/Fest/Flüssig-Systeme wie Suspensions-/Emulsionssysteme. Dem Fachmann sind verschiedene Möglichkeiten bekannt, derartige Träger bereitzustellen. Bei den O/W-Emulsionen sind bevorzugte galenische Leitsubstanzen O/W-Emulgatoren, W/O-Emulgatoren, flüssige hydrophile Bestandteile und flüssige lipophile Bestandteile. Bei den W/O-Emulsionen sind bevorzugte galenische Leitsubstanzen W/O-Emulgatoren, O/W-Emulgatoren, flüssige und halbfeste lipophile Bestandteile, Gelbildner, flüssige hydrophile Bestandteile und/oder Salze.

Bei den halbfesten bevorzugten Trägersubstanzen sind wasserfreie Systeme wie wasserhaltige Systeme für unterschiedliche Anwendungen bevorzugt. Wasserfreie Systeme können aus apolaren Systemen bestehen oder aus polaren Systemen ohne Emulgatoren wie Lipogele, Oleogele oder Polyethylenglycolgele bzw. aus apolaren Systemen mit Emulgatoren auf O/W-Absorptionsgrundlagen oder W/O-Absorptionsgrundlagen. Die wasserhaltigen Systeme können bevorzugt aus einphasigen Systemen wie Hydrogelen oder Mikroemulsionsgelen oder mehrphasigen Systemen wie O/W-Cremes, W/O-Cremes oder amphiphilen Systemen bestehen. Die bevorzugten halbfesten Zubereitungen sind im Temperaturbereich zwischen Raumtemperatur und Hauttemperatur streichfähige Zubereitungen zur Anwendung auf der Haut oder auf Schleimhäuten, die eine lokale Wirkung ausüben, Wirkstoffe transportieren oder eine erweichende oder schützende Wirkung auf die Haut ausüben. Bevorzugte Zubereitungen sind Salben im engeren Sinne, Cremes, Gele und/oder Pasten. Neben den Salben, Cremes, Gelen und Pasten können auch Oleogele als halbfeste, transparente Einphasensysteme eingesetzt werden. Dem Fachmann sind beispielsweise aus dem US-Patent 6,187,323 oder Aiache et al. 2001 verschiedene wasserfreie Compounds zur Formulierung halbfester Systeme bekannt, wie beispielsweise die Verbindung aus einem Oleogel und eine Hydrogel, welche erfindungsgemäß als Bi-Gel bezeichnet werden kann. Weiterhin können Hydrodispersionsgele oder verschiedene Lipide herangezogen werden, um erfindungsgemäße Trägersubstanzen bereitzustellen. Beim Einsatz von Lipiden können siliziumorganische Verbindungen ebenso wie kohlenstofforganische Verbindungen zur Bereitstellung von Lipidphasen in dispersen Systemen zum Einsatz kommen, wobei kohlenstofforganische Verbindungen beispielsweise mithilfe von nicht-hydrolisierbaren Lipiden oder hydrolisierbaren Lipiden (Glyzerine), Wachsester bereitgestellt werden können. Vorteile derartiger Systeme sind die verbesserte Geschmeidigkeit der Haut und die Steigerung ihrer Elastizität sowie ihre Fähigkeit, je nach Zusammensetzung der Lipide freisetzungs- und penetrationsbeeinflussend wirken zu können. Dem Fachmann ist bekannt, welche Lipide er einsetzen muss, um beispielsweise die Penetration innerhalb eines Zeitparameters zu steigern oder zu vermindern.

Weitere bevorzugte Trägersubstanzen sind beispielsweise Hydrodispersionsgele bzw. Mikrokapseln, Mikrosphärulen oder Pellets (Macrobeads). Die genannten Träger dienen der Stabilitätserhöhung und stellen eine Mindestanwendungsdauer auf der Haut sicher. Die bevorzugten halbfesten Einphasensysteme können mithilfe der folgenden galenischen Leitsubstanzen bereitgestellt werden: flüssige hydrophile Bestandteile, insbesondere Wasser und (Poly-)Alkohole, hydrophile Gelbildner, Salzbildner sowie W/O-Emulgatoren, O/W-Emulgatoren, flüssige, halbfeste und feste lipophile Bestandteile sowie lipophile Gel- bzw. Gerüstbildner. Dem Fachmann ist bekannt, wie er diese Substanzen kombinieren muss, um eine bestimmte Wirkung zu erzielen.

Dem Fachmann sind weitere galenische Zubereitungen für dermale Produkte bekannt. Anmeldungsgemäß können beispielsweise alle in Daniels and Knie in der JDDG; 2007, 5: 367-383 offenbarten galenischen Verbindungen eingesetzt werden. Dem Fachmann ist bekannt, dass unterschiedliche galenische Zubereitungen unterschiedlich auf der Haut wirken und die erfindungsgemäße Zusammensetzung unterschiedlich stark in die Haut eintragen. Der Inhalt der JDDG; 2007, 5: 367-383 ist in den Offenbarungsgehalt der anmeldungsgemäßen Lehre mit aufgenommen. Bevorzugte erfindungsgemäße Produkte sind beispielsweise lipophile oder hydrophile Lösungen, lipophile oder hydrophile Emulsionen, lipophile oder hydrophile Suspensionen, spezielle flüssige Zubereitungen, hydrophobe oder hydrophile Salben, Wasser-emulgierende Salben, lipophile, hydrophile oder amphiphile Cremen, Hydrogele, hydrophobe oder hydrophile Pasten und/oder Puder.

Die bevorzugten Milchsäurebakterien sind durch den funktionellen Zusammenhang dergestalt miteinander zu einer einheitlichen erfinderischen Idee verbunden, dass sie Eigenschaften bzw. Wirkungen gemein haben, nämlich dass sie spezifisch das pathogene Bakterium *Streptococcus pyogenes* koaggregieren, keine kommensale Mikroorganismen der Haut bzw. Schleimhäute binden und zudem die Bindung an Fibronectin durch *Streptococcus pyogenes* verhindern bzw. die an Fibronectin gebundenen *Streptococcus pyogenes* Zellen zerstören. Die genannten Milchsäurebakterien umfassen insbesondere Mikroorganismus ausgewählt aus der Gruppe umfassend folgende bei der Deutschen Sammlung für Mikroorganismen und Zellkulturen hinterlegten als DSM 25972, DSM 25987, DSM 25988, DSM 25989, DSM 25973 nummerierten Mikroorganismen. Es war völlig überraschend, dass eine Gruppe Milchsäurebakterien identifiziert werden konnten, die identische vorteilhafte Eigenschaften aufweisen. Im Stand der Technik sind keine Bakterien, insbesondere Milchsäurebakterien beschrieben, die alle Eigenschaften vereinen und zudem apathogen sind und zu keiner Schädigung oder Beeinflussung der natürlichen Hautflora führen. Es hat sich zudem gezeigt, dass eine Applikation der bevorzugten Milchsäurebakterien, sei es als Zusammensetzung oder anderweitig, die Bindung und Invasion von Wirtszellen durch *Streptococcus pyogens* verhindert. Die Ursache hierfür ist bis jetzt unbekannt, soll aber durch weitere Versuche identifiziert werden.

Ein weiterer überraschender Vorteil der bevorzugten Milchsäurebakterien ist, dass diese auch prophylaktisch verwendbar sind. Das heißt, die mit Milchsäurebakterien, bzw. eine diese umfassende Zusammensetzung können prophylaktisch appliziert werden (vorzugsweise in Form von Lutschpastillen oder Bonbons), ohne dass hierdurch eine Schädigung der Haut- und Rachenschleimhaut oder der autochthonen Flora erfolgt.

Im Sinne der Erfindung wird die Haut bzw. Schleimhaut in bevorzugten Ausführungsformen insbesondere als äußeres Organ des menschlichen oder tierischen Organismus verstanden, welches der Abgrenzung von innen und außen dient. Zu einem Hautareal im Sinne der Erfindung gehören in bevorzugten Ausführungsformen Bestandteile der Schleimhäute.

Die Verträglichkeit der Milchsäurebakterien der Haut und Schleimhäute ist eine die Voraussetzung für eine erfolgreiche Behandlung von bakteriell verursachten Entzündungen, Erkrankungen oder Beschwerden im Hals- und Rachenbereich, bei denen Mikroorganismen aus der Gruppe *Streptococcus* vorkommen. Die bevorzugte Zusammensetzung soll insbesondere als Probiotikum als Lutschpastille, Bonbon oder Dragee, Saft oder Sirup, Spüllösung, Spray oder Aerosol eingebracht werden, um die Bildung des pathogenen Leitkeimes und dessen Bindung an Wirtszellen zu unterbinden, diesen zu binden, diesen als Aggregat zu entfernen und/oder ihn zu hemmen bzw. abzutöten und ihn so zu reduzieren.

Die Anwendung der erfindungsgemäßen Zusammensetzung erfolgt bevorzugt als Bonbon, Lutschpastille oder Dragee. Zum einen ermöglicht die Formulierung als Bonbon, Lutschpastille oder Dragee eine in der Regel wohlschmeckende Rezeptur (u.a. Zugabe von Zuckern, Zuckeraustauschstoffe, Aromen), die bei Patienten und Verbrauchern große Akzeptanz findet. Zum anderen befeuchtet der beim Lutschen zusätzlich gebildete Speichel die Schleimhäute, so dass bei Symptomen einer Hals- oder Mandelentzündung eine Linderung erfahren wird. Beim Lutschen von Bonbons, Lutschpastillen oder Dragees wird die erfindungsgemäße Zusammensetzung langsam und über einen längeren Zeitraum freigesetzt, was für den Aspekt der Anwendung von Vorteil ist. Zudem ist eine Einarbeitung der erfindungsgemäßen Zusammensetzung in eine Bonbon-, Pastillen-, oder Drageemasse technisch sehr gut umsetzbar. Diesbezüglich war es völlig überraschend, dass Milchsäurebakterien bereitgestellt werden konnten, die eine Aktivität bei einer Körpertemperatur aufweisen, aber auch nach eine Hitzebehandlung bei 70° bis 90° Celsius derart durchstehen, dass sie auch nach der Behandlung die bevorzugten Fähigkeiten, insbesondere die Koaggregation von *Streptococcus pyogenes* aufweisen. Dies ist insbesondere deswegen vorteilhaft, da bei der Herstellung von Bonbons nicht selten Temperaturen von 70° Celsius erreicht werden, so dass es für die Verwendung der Milchsäurebakterien oder der Zusammensetzung in Form eines Bonbons insbesondere vorteilhaft ist, dass die Milchsäurebakterien auch noch nach der Herstellung der Bonbons die Koaggregationsfähigkeit besitzen. Aus dem Stand der Technik ist nichts dergleichen bekannt. Die bevorzugten Milchsäurebakterien besitzen somit Fähigkeiten, die sie deutlich vom Bekannten abgrenzen. Weiterhin war es überraschend, dass die Bonbons, die die Zusammensetzung mit den Milchsäurebakterien aufweisen, sich langsamer im Mund auflösen und somit der Bonbon eine langandauernde und effektivere Wirkung erzielt. Dies war völlig überrachend für die Erfinder. Außerdem haben Versuche gezeigt, dass insbesondere durch die langsame Freisetzung der Milchsäurebakterien im Wesentlichen alle *Streptococcus pyogenes* Zellen im Mundraum koaggregiert werden können.

Dem Fachmann ist bekannt, was unter dem Begriff "Körpertemperatur" zu subsumieren ist und wie diese bestimmt werden kann. Hierbei bezieht sich die Körpertemperatur im Wesentlichen auf Tiere und den Menschen. Dem Fachmann ist ferner der Begriff "Bonbon" bekannt und er weiß zudem, in welcher Standardliteratur er Bestandteile oder die Herstellung eines Bonbons nachschlägt. Die Erfindung beschränkt sich nicht auf einfache Bonbons, sondern im Wesentlichen kann die Erfindung mit allen Bonbons kombiniert werden, die dem Fachmann bekannt sind. Bonbons und deren Herstellung sind dem Fachmann in entsprechender frei zugänglicher Fachliteratur offenbart, z. B. in Candy Ind. 27, 161 (1996); Hoffmann, H.; Mauch, W.; Untze, W., Zucker und Zuckerwaren, 2. Auflage; Behr's: Hamburg, (2002); Lees, R.; Jackson, B., Sugar Confectionery and Chocolate Manufacture, Leonard Hill Books: Plymouth, (1999); Bundesverband der Deutschen Süßwarenindustrie e. V. (BDSI), Hrsg., Süßwarentaschenbuch, Molberg: Bonn, (2001); Taschenbuch für Lebensmittelchemiker; Lebensmittelführer, Thieme: Stuttgart, (1995).

Bonbons können in einer Vielfalt an Formen, Geschmacksrichtungen, Konsistenzen und Farben bevorzugt sein. In der Regel handelt es sich um bissengroße Stücke. Bonbons können massiv oder gefüllt sein, rund, oval, quader- oder würfelförmig. Je nach Restwassergehalt sind sie hart (Hartkaramellen) oder weich und kaubar (Weichkaramellen). Nach den Zutaten unterscheidet man insbesondere zwischen Hals- und Husten-, Frucht-, Karamell- und Erfrischungsbonbons. Wirkung und Geschmack der Bonbons kann durch den Einsatz von Kräuterextrakten, ätherischen Ölen (wie zum Beispiel Eukalyptus) und Wirkstoffen wie Menthol bestimmt werden. Bei Fruchtbonbons können Aromastoffe und Säureanteile einen individuellen Geschmack erzeugen. Natürliche Frucht- und Pflanzenextrakte oder auch Lebensmittelfarbstoffe können zudem hinzugefügt werden, um den Bonbons ihre Färbung zu geben. Karamellbonbons werden insbesondere aus Milchprodukten wie Kondensmilch, Butter und Sahne hergestellt. Mittels Aromatisierung und diverser Zutaten sind Geschmacksvarianten wie Nuss, Mandel, Honig, Kakao, Kokos und ähnliches möglich. Es sind zudem Kaubonbons, geschnittene, geprägte, gegossene oder kaschierte Bonbons bevorzugt. Vorteilhafte Bonbons können mit unterschiedlichen Inhaltsstoffen versehen werden. So können z. B. Vitamine, Aminosäuren, Öle, Kräuter, Fette, unterschiedliche Kohlenhydrate oder Zuckerersatzstoffe, Geschmacksverstärker und/oder Emulgatoren umfassen. Hartkaramelle weisen insbesondere Saccharose und Glucose auf, die bevorzugt unter Verwendung von geruch- und geschmackgebenden, färbenden und die Beschaffenheit beeinflussenden Stoffen hergestellt werden. Aufgrund des niedrigen Restwassergehalts von 1-3 % besitzen Hartkaramelle eine harte, oft glasartige Konsistenz. Hartkaramelle können auch zuckerfrei unter Verwendung von Süßstoffen und Zuckeraustauschstoffen hergestellt werden.

Weichkaramelle bezeichnen insbesondere eine Gruppe von Zuckerwaren, die im Gegensatz zu Hartkaramellen eine kaubare Konsistenz haben und die bei einem Wassergehalt von 6-10 % aus den Hauptbestandteilen Saccharose, anderen Zuckerarten, Süßungsmitteln und/oder Zuckeralkoholen sowie Glucosesirup durch satzweises oder kontinuierliches Einkochen hergestellt werden und denen bevorzugt Fett, Milchbestandteile, Dickungsmittel, Emulgatoren, geruch- und geschmacksgebende Stoffe beigemischt sind. Sie können ebenfalls in den verschiedensten Formen und Farben, mit und ohne Füllung hergestellt werden.

Weiterhin hat es sich als vorteilhaft herausgestellt, wenn die Bonbons ein Mehrkammersystem aufweisen, wobei in einer Kammer Vitamine oder sonstige Co-Faktoren und in einer oder mehreren weiteren Kammern die bevorzugten Milchsäurebakterien oder Zusammensetzung vorliegen. Durch dieses Kammersystem kann sichergestellt werden, dass die Milchsäurebakterien zu einem optimalen Zeitpunkt und ggf. in unterschiedlichen Dosen freigesetzt werden. Die Vitamine oder sonstige Co-Faktoren können zudem die Aktivität der Milchsäurebakterien fördern. Beispielsweise hat sich überraschenderweise gezeigt, dass die Moosbeere (auch bekannt als Cranberry) biofilmverhindernd wirkt und sich somit optimal mit den bevorzugten Milchsäurebakterien ergänzt. Versuche haben gezeigt, dass die Koaggregation von *Streptococcus pyogenes* um 50% effizienter abläuft, wenn Bestandteile oder eine Lösung der Cranberry vorhanden sind.

Versuche haben gezeigt, dass die bevorzugten Milchsäurebakterien beim Kontakt mit *Streptococcus pyogenes* Zellen Koaggregate ausbilden. Durch die Bildung von Koaggregaten wird *Streptococcus pyogenes* daran gehindert, insbesondere Zellen des Hals- und Rachenbereiches zu besiedeln und zu invadieren. Die *Streptococcus pyogenes* Zellen, insbesondere deren Zelloberfläche, werden durch die Milchsäurebakterien, insbesondere Lactobacillus Zellen maskiert, so dass die *Streptococcus pyogenes* Zellen bevorzugt nicht mehr in der Lage sind, an die Haut- und Schleimhautepithelzellen zu binden. Durch die verhinderte Bindung von *Streptococcus pyogenes* an die Haut- und Schleimhautepithelzellen bleiben Entzündungsreaktionen aus. Es war völlig überraschend, dass Milchsäurebakterien bereitgestellt werden konnten, die zu einem sehr frühen Stadium mit *Streptococcus pyoge*nes koaggregieren und somit eine Bindung von *Streptococcus pyogenes* an Epithelzellen von Vorneherein verhindert. Dies war völlig überraschend und ist nicht aus dem Stand der Technik bekannt. Die bevorzugten Milchsäurebakterien können somit bereits im Mundraum mit *Streptococcus pyogenes* koaggregieren. Außerdem war es völlig überraschend, dass die bevorzugten Milchsäurebakterien nicht durch die im Speichel auftretenden Enzyme oder sonstigen Faktoren inhibiert, d. h. in ihrer Fähigkeit zur Koaggregation von *Streptococcus pyogenes* gehemmt werden.

Natürlicher Speichel enthält u. a. antimikrobielle Substanzen, wie beispielsweise Lysozym, das der Abwehr von Bakterien dient, und das die Zellwand von insbesondere Gram-positiven Bakterien direkt angreifen kann. Die bevorzugten Milchsäurebakterien zeichnen sich jedoch dadurch aus, dass sie trotz des Vorliegens von natürlichem Speichel und trotz der darin enthaltenen antimikrobielle Stoffe immer noch zur spezifischen Koaggregation von *Streptococcus pyogenes* befähigt sind. Dies war völlig überraschend und stellt einen erheblichen Vorteil gegenüber dem Stand der Technik dar.

Im Sinne der Erfindung umfassen probiotische Mikroorganismen Zellen, welche vorteilhafte Wirkungen auf menschliche und/oder tierische Körper zeigen. Eine bevorzugte Zusammensetzung wird als probiotische Zusammensetzung verwendet und enthält, Milchsäurebakterien, die eine vorteilhafte Wirkung auf menschliche und/oder tierische Körper zeigen. Vorteilhafte Wirkungen können insbesondere in der Reduktion der Keimbelastung von Hals- und Rachenbereich durch *Streptococcus pyogenes* bestehen. Insbesondere können in der autochthonen Flora unerwünschte Mikroorganismen, wie *Streptococcus pyogenes* durch unmittelbare Wechselwirkungen mit den probiotischen Mikroorganismen und dem unerwünschten Mikroorganismus und insbesondere, durch mittelbare Wechselwirkungen auf Grund von Hemmungen des Metabolismus des unerwünschten Mikroorganismus durch Expressionsprodukte des probiotischen Mikroorganismus gehemmt werden. Es konnte durch Versuche gezeigt werden, dass der pathogene Keim *Streptococcus pyogenes* nach einer Koaggregation durch die bevorzugten Milchsäurebakterien kein Wachstum, d. h. keine Vermehrung der Zellmasse mehr aufweisen, sondern die Zellen insbesondere maskiert und/oder abgetötet werden.

Außerdem war es überraschend, dass die Milchsäurebakterien auch nach physikalischer, chemischer und/oder biologischer Abtötung die vorteilhaften Eigenschaften besitzen. Beispielsweise konnten die bevorzugten Stämme, nämlich DSM 25972, DSM 25987, DSM 25988, DSMZ 25989 und DSM 25973, auch nach Hitzebehandlung bei 70° Celsius für 20 Minuten oder einer Behandlung mit Ultraschall eine Koaggregation des pathogenen Keimes bewirken, die Bindung an Fibronectin verhindern und zudem keine Bindung kommensaler Mikroorganismen zeigen. Somit können die Milchsäurebakterien vorteilhafterweise auch abgetötet in einer bevorzugten Ausführung der Zusammensetzung vorliegen. Hierdurch kann die Haltbarkeit und Nutzbarkeit der Zusammensetzung erheblich verlängert werden. Außerdem kann die Zusammensetzung in weiteren Anwendungsbereichen Verwendung finden, die die Nutzung von lebenden Mikroorganismen nicht zulassen, hergestellt werden durch thermische Abtötung oder Lyophilisation, insbesondere durch biologische, chemische oder physikalische Abtötungsverfahren, wobei das Analogon oder Fragment auch nach Abtötung oder nach Sprühtrocknung die Fähigkeit zur spezifischen Bindung der pathogenen Mikroorganismen aufweist, welcher aus der Gruppe *Streptococcus pyogenes* ausgewählt ist. Es war völlig überraschend, dass die Milchsäurebakterien in der Zusammensetzung lebend oder nicht-lebend (tot) vorliegen und nichtsdestotrotz den pathogenen Mikroorganismus spezifisch binden, bzw. diesen koaggregieren.

Ferner betrifft die vorliegende Erfindung auch Zusammensetzungen, die das erfindungsgemäße Milchsäurebakterium enthalten, welche die Fähigkeit zur spezifischen Bindung des oben aufgeführten pathogenen Mikroorganismus aufweisen, und die insbesondere als Nahrungsmittel, Nahrungsmittelzusatz, als Tiernahrung oder Getränke eingesetzt werden. Es ist eine Zusammensetzung bevorzugt, die die erfindungsgemäßen Milchsäurebakterien enthält, welche die Fähigkeit zur spezifischen Bindung bzw. Koaggregation mindestens eines pathogenen Mikroorganismus aus der Gruppe *Streptococcus pyogenes* aufweisen, wobei die Zusammensetzung für die Behandlung oder Prophylaxe von Erkältungserkrankungen verwendet wird.

Die erfindungsgemäßen bevorzugten Mikroorganismen sind Vertreter der Gattung der Milchsäurebakterien, mithin also Gram-positive Bakterien, die Milchsäure durch Fermentation von Glucose erzeugen. Die erfindungsgemäßen Milchsäurebakterien zeichnen sich einerseits dadurch aus, dass sie die Fähigkeit zur spezifischen Bindung Koaggregation von zumindest einem pathogenen Mikroorganismus aufweisen, der ausgewählt ist aus der Gruppe Streptococcus pyogenes. Diese Bindung führt zu einer Bildung von Aggregaten aus den erfindungsgemäßen Milchsäurebakterien und den spezifisch gebundenen, pathogenen Mikroorganismen. Durch die Aggregat-Bildung können letztere, also die pathogenen Mikroorganismen mechanisch, bspw. durch Abspülen, gezielt und leicht entfernt werden, was durch bisher bekannte Maßnahmen nicht möglich war.

Unter dem Ausdruck "spezifische Bindung" oder "Koaggregation" wird im Sinne der Erfindung sowie üblicherweise im Gebiet der Mikrobiologie und Hygiene, insbesondere der humanen Mikrobiologie und Körperhygiene die gegenseitige Erkennung und Adhäsion von Zellen, die genetisch unterschiedlichen Typen von Zellen angehören, verstanden. Koaggregation wird im Sinne der Erfindung insbesondere als Adhäsion, Interaktion, Bindung, spezifische Bindung, Affinität oder Wechselwirkung bezeichnet und kennzeichnet insbesondere die Fähigkeit der bevorzugten Milchsäurebakterien, mit *Streptococcus pyogenes* Agglomerate zu bilden. Hierzu exprimieren Bakterien auf ihrer Zelloberfläche Rezeptoren und Strukturen für Adhäsine auf anderen Zelltypen, die für die Anheftung zwischen den Zellen verwendet werden. Diese Adhärenz spielt für die Besiedelung mit pathogenen wie auch mit kommensalen Mikroorganismen eine herausragende Rolle, so dass ein Eingriff in die Adhärenz zu weitreichenden Konsequenzen führen kann. Dadurch, dass die erfindungsgemäßen Milchsäurebakterien die Fähigkeit zur spezifischen Bindung, insbesondere und Koaggregation mit zumindest einem Mikroorganismus der Gruppe *Streptococcus pyogenes* aufweisen, können sich Aggregate aus den erfindungsgemäßen Milchsäurebakterien und den pathogenen Mikroorganismen bilden. Die entstehenden Koaggregate können leicht, bspw. durch Spülen, von Oberflächen, der Haut, Geweben und/oder einem sonstigen Besiedlungsort oderreservoir entfernt werden, so dass die Zahl der pathogenen Mikroorganismen deutlich reduziert wird. Des Weiteren wird durch Maskierung der Oberflächenstrukturen der pathogenen Mikroorganismen eine initiale bzw. erneute Anheftung an Oberflächen, der Haut, Geweben und/oder sonstigen Besiedlungsorten oder -reservoirs verhindert bzw. reduziert. Wenn Zellen untereinander binden und Agglomerate bilden, bezeichnet man diesen Vorgang insbesondere als Aggregation. Wenn bei dieser Aggregat-Bildung nur eine Zellart beteiligt ist, wird das als Autoaggregation oder Selbstaggregation bezeichnet. Sind bei der Aggregat-Bildung mindestens zwei verschiedene Zellarten beteiligt, bezeichnet man den Vorgang insbesondere als Koaggregation.
Im Sinne der Erfindung wird mit "spezifische Bindung von zumindest einem pathogenen Mikroorganismus" insbesondere die Eigenschaft der erfindungsgemäßen Milchsäurebakterien verstanden, mindestens ein Bakterium aus der Gruppe umfassend *Streptococcus pyogenes* spezifisch zu binden.
Gemäß einer bevorzugten Ausführungsform der erfindungsgemäßen Milchsäurebakterien zeichnen sich diese ferner dadurch aus, dass die Fähigkeit zur spezifischen Bindung an zumindest einen pathogenen Mikroorganismus auch nach einer biologischen, chemischen oder physikalischen Behandlung, z. B. einer Hitzebehandlung bei mind. 70 °C besteht. Das heißt, die bevorzugten Milchsäurebakterien können bevorzugt abgetötet in der einer bevorzugten Zusammensetzung vorliegen, da die Fähigkeit zur spezifischen Wechselwirkung oder Bindung mit pathogenen Bakterien, insbesondere *Streptococcus pyogenes* nicht beeinflusst ist und somit eine Bindung oder Wechselwirkung hergestellt werden kann.
Unter der genannten Hitzebehandlung bzw. unter "resistent gegenüber einer Hitzebehandlung" oder "Hitzestabilität" wird dabei die Eigenschaft eines Bakteriums verstanden, auch nach einem bestimmten, längeren Zeitraum von mindestens 30 min bei einer erhöhten Temperatur von 70 °C oder mehr noch in der Lage zu sein, eine spezifische Bindung mit zumindest einem pathogenen Mikroorganismus einzugehen, das aus der Gruppe *Streptococcus pyogenes* ausgewählt ist. Abgetötete oder tote Milchsäurebakterien Zellen können besonders vorteilhaft sein, da keine metabolische Aktivität von den Milchsäurebakterien Zellen ausgehen kann. "Abgetötete", "tote" oder "getötete" Formen werden im Sinne der Erfindung insbesondere dadurch charakterisiert, dass diese Formen der erfindungsgemäßen Milchsäurebakterien z.B. nicht mehr teilungsfähig sind, mit Lebendfarbstoffen nicht anfärbbar sind, metabolisch nicht aktiv sind sowie keine DNA Replikation oder Proteinbiosynthese oder Sekretion, z.B. von Stoffwechselprodukten, aufweisen.
Gemäß einer weiteren bevorzugten Eigenschaft der Milchsäurebakterien können sich die erfindungsgemäßen Milchsäurebakterien zusätzlich zu der oben beschriebenen Eigenschaft der Fähigkeit zur spezifischen Bindung mindestens eines pathogenen Mikroorganismus, der aus der Gruppe *Streptococcus pyogenes* ausgewählt ist, auch durch eine Hitzestabilität auszeichnen, und eine Behandlung mit hohen Temperaturen, vorzugsweise von mindestens etwa 60 °C, bevorzugter von mindestens 65 °C und noch bevorzugter von mindestens 70 °C, über einen Zeitraum von mindestens 20 Minuten, vorzugsweise von 25 Minuten und noch bevorzugter von mindestens ca. 30 Minuten, überstehen und bezüglich ihrer Fähigkeit zur Koaggregation spezifischen Bindung von genanntem pathogenen Mikroorganismus unverändert bleiben.

In einer bevorzugten Ausführungsform der Zusammensetzung sind somit Milchsäurebakterien bevorzugt, die lebend oder abgetötet sind oder Teile und Fragmente, z.B. enzymatische oder mechanische Spaltprodukte (z.B. French Press etc.) oder Stoffwechselprodukte dieser, soweit diese noch die Fähigkeit zur Koaggregation und/oder der Verhinderung der Bindung an Fibronectin aufweisen. Es ist zudem bevorzugt, dass die Milchsäurebakterien in verkapselter, sprühgetrockneter und/oder lyophilisierter Form eingesetzt werden, d. h. in verkapselter, sprühgetrockneter und/oder lyophilisierter Form in einer bevorzugten Zusammensetzung vorliegen. Außerdem kann es vorteilhaft sein, wenn die Milchsäurebakterien in Form eines Zellaufschlusses eingesetzt werden.

Ferner ist bevorzugt, wenn die Fähigkeit der erfindungsgemäßen Milchsäurebakterien zur spezifischen Bindung an die pathogenen Mikroorganismen auch bei einem pH-Wert von zwischen ca. 3 bis 8 besteht. Das heißt, die Milchsäurebakterien können sich in einem Milieu befinden, welches einen pH-Wert von 3 bis 8 aufweist und noch ihre Fähigkeit zur Bindung von *Streptococcus pyogenes* aufweisen. Die bevorzugten Milchsäurebakterien können vorteilhafterweise in einem großen pH-Wert-Bereich eingesetzt werden, bzw. zeigen in dem bevorzugten Bereich die bevorzugten Eigenschaften. Die bevorzugten Milchsäurebakterien können somit universell in unterschiedlichen Körperbereichen eingesetzt werden. Überraschenderweise zeigen die erfindungsgemäßen Milchsäurebakterien in einem breiten Temperaturbereich von ca. 25°C - 42° C Koaggregatonseigenschaften insbesondere mit *Streptococcus pyogenes.* Dem Fachmann wird hierbei, sowie bei sämtlichen in der vorliegenden Erfindung aufgeführten Bereichsangaben, die durch die Begriffe "ca. " oder "etwa" gekennzeichnet sind, klar sein, dass mit dem Ausdruck "ca. " oder "etwa" nicht die exakte Zahlenangabe gemeint sein muss, sondern dass auch geringfügige Abweichungen nach oben oder unten bezüglich der angegebenen Zahl noch in den Rahmen der vorliegenden Erfindung liegen. Die Bindung der erfindungsgemäßen Milchsäurebakterien an die aufgeführten pathogenen Mikroorganismen führt bevorzugt zu einer Hemmung des Wachstums des pathogenen Mikroorganismus.

Es war überraschend, dass durch die Bindung des pathogenen Mikroorganismus und den erfindungsgemäßen Milchsäurebakterien ein Aggregat gebildet wird, das sich bei einer Zentrifugalkraft von bevorzugt 300 x g über insbesondere 10 Sekunden abzentrifugieren lässt und insbesondere nach bevorzugt 10 min Inkubation bei Raumtemperatur ohne Agitation als Sediment vorliegt.

Gemäß einer weiteren bevorzugten Ausführungsform besitzen die erfindungsgemäßen Milchsäurebakterien nicht die Fähigkeit, an kommensale Hals- und Rachenflora, wie *Streptococcus salivarius* zu binden, wobei insbesondere *Streptococcus salivarius* ein weitestgehend unauffälliger Kommensale der Hautflora darstellt. Im Zusammenspiel der unterschiedlichen, in oder auf der Haut vorhandenen Mikroorganismen ist er in der gesunden Hautflora vieler Säugetiere vorhanden, und steht - zumindest bei gesunder Haut - mit diesen im mikrobiellen Gleichgewicht. Daher ist eine Beeinflussung dieses Bakteriums, bspw. durch eine Aggregation und dadurch Beeinflussung mit anderen Mikroorganismen, die gezielt in der Körperhygiene eingesetzt werden sollen, im Rahmen der vorliegenden Erfindung nicht bevorzugt.

Die vorliegende Erfindung betrifft in einer bevorzugten Ausführungsform Milchsäurebakterien, wobei die in einer bevorzugten Ausführungsform bevorzugten Milchsäurebakterien zumindest eines der folgenden Merkmale aufweist: a) Hitzestabilität oder stabil nach biologischer, und/oder chemischer und/oder physikalischer Behandlung oder b) Fähigkeit zur Hemmung der Adhäsion von Wirtszellen durch *Streptococcus pyogenes.* Es war völlig überraschend, dass die erfindungsgemäßen Milchsäurebakterien auch nach biologischer, und/oder chemischer und/oder physikalischer Behandlung ihre Fähigkeiten erhalten, *Streptococcus pyogenes* spezifisch zu binden.

Eine weitere Eigenschaft der erfindungsgemäßen Milchsäurebakterien ist die Fähigkeit zur Hemmung der Bindung an Wirtszellen durch *Streptococcus pyogenes,* indem diese bereits als planktonisch vorliegende Zellen spezifisch koaggregiert und als Aggregate weggespült werden. Aufgrund der Koaggregation können diese pathogenen Bakterien biologische Oberflächen nicht mehr besiedeln, invadieren und können konsequenterweise auch keine Erkrankungen auslösen.

Daher wird im Sinne der Erfindung insbesondere unter" Hemmung der Bindung an Wirtszellen (Hals- oder Rachenzellen) durch *Streptococcus pyogenes"* jede Eigenschaft der erfindungsgemäßen Milchsäurebakterien verstanden, derart mit Streptococcus pyogenes zu interagieren, d.h. an diese zu binden oder sie anderweitig zu beeinflussen, so dass diese nicht mehr an Hals- oder Rachenzellen binden können.

Gemäß einer bevorzugten Ausführungsform kann das erfindungsgemäße Milchsäurebakterium daher mindestens eine der aufgeführten Eigenschaften Resistenz gegenüber biologischer, chemischer und/oder physikalischer Behandlung, Hitzeresistenz, Fähigkeit zur spezifischen Bindung, Koaggregation an *Streptococcus pyogenes* aufweisen. Bevorzugte Kombination der Eigenschaften sind z. B. oder aber zwei, also bspw. Resistenz gegenüber biologischer, chemischer und/oder physikalischer Behandlung Hitzeresistenz und Fähigkeit zur Bindung an Streptococcus pyogenes, oder Resistenz gegenüber biologischer, chemischer und/oder physikalischer Behandlung Hitzeresistenz und Hemmung der Bindung von Hals- oder Rachenzellen durch *Streptococcus pyogenes* aufweisen.

Vorzugsweise handelt es sich bei den erfindungsgemäßen Milchsäurebakterien um Bakterien der Art *Lactobacillus paracasei subsp. paracasei, Lactobacillus plantarum, Lactobacillus crispatus* und *Lactobacillus ingluviei.*

Entsprechend besitzt "eine Mutante oder ein Derivat" der Milchsäurebakterien, insbesondere eine Mutante oder ein Derivat der im Rahmen der vorliegenden Anmeldung hinterlegten Lactobacilli sp., und die gleichen Charakteristika wie vorliegend für die beanspruchten Milchsäurebakterien beansprucht, und insbesondere die gleichen wie die hinterlegten Stämme; dies wären zumindest die Fähigkeit zur spezifischen Bindung, Koaggregation mindestens eines pathogenen Mikroorganismus ausgewählt aus der Gruppe *Streptococcus pyogenes.* Weiterhin ist bevorzugt und bevorzugt mindestens eines der folgenden Merkmale (i) Resistenz der Fähigkeit zur spezifischen Bindung gegenüber einer biologischen, chemischen und/oder physikalischen Behandlung, insbesondere einer Hitzebehandlung bei mehr als 70 °C für mindestens 30 min; (ii) keine Bindung an *Streptococcus salivarius*; (iii) Fähigkeit zur spezifischen Bindung an *Streptococcus pyogenes*; (iv) Fähigkeit zur Hemmung der Bindung an Hals- oder Rachenzellen durch *Streptococcus pyogenes*; (v) Zustandekommen der spezifischen Bindung bei pH 3 - 8 (vi) Fähigkeit zur spezifischen Bindung in natürlichem Speichel. Solche Bevorzugte Derivate können bspw. gentechnologisch hergestellt werden. Dabei beinhaltet der Ausdruck "genetechnologisch hergestellt" im Sinne der Erfindung insbesondere alle Verfahren, die dem Fachmann im Gebiet der Gentechnologie bekannt sind, um Nukleinsäuren in vitro und in vivo derart zu modifizieren, dass durch rekombinante DNA-Technologien genetische Modifizierungen bewirkt und Gene verändert werden können.

Ein "Fragment" im Sinne der vorliegenden Erfindung ist insbesondere jeder zellulärer Bestandteil der erfindungsgemäßen Milchsäurebakterien und vorzugsweise ein Teil der Zellmembran. Die Gewinnung von Zellmembran-Fraktionen ist dem Fachmann aus dem Stand der Technik hinreichend bekannt.

Die erfindungsgemäßen Milchsäurebakterien liegen dabei vorzugsweise isoliert oder gereinigt vor, wobei der Begriff "isoliert" insbesondere bedeutet, dass die Milchsäurebakterien ihrem Kultivierungsmedium/-milieu - auch bspw. ihrem natürlichen - entnommen sind. Der Ausdruck "gereinigt" ist nicht auf eine absolute Reinheit beschränkt.

Es ist bevorzugt, dass neben den erfindungsgemäßen Milchsäurebakterien in lebensfähiger Form auch abgetötete Formen der erfindungsgemäßen Milchsäurebakterien vom Rahmen der vorliegenden Erfindung umfasst sind. Geeignete Verfahren zur Abtötung (z. B. biologische, chemische oder, physikalische Abtötungsmethoden) sind dem Fachmann hinreichend bekannt. Vorliegend können aber die Milchsäurebakterien auch in lyophilisierter Form eingesetzt werden. Lyophilisierte Zellen können nach geeigneter Kultivierung in flüssigen oder festen Medium wieder zum anwachsen gebracht werden. Dies war völlig überraschend und betrifft eine neue und erfinderische Nische. Im Stand der Technik sind z. B. Lösungen zur Reinigung von Oberflächen bekannt, die Sporen beinhalten, wobei die Bakterien reaktiviert werden, wenn die Sporen mit entsprechenden Substraten in Kontakt kommen. Für die bevorzugten Milchsäurebakterien sowie die Zusammensetzung ist nichts dergleichen notwendig. Die bevorzugten Milchsäurebakterien sind auch aktiv, d. h. koaggregieren an *Streptococcus pyogenes* und weisen auch andere zuvor beschriebene Eigenschaften auf, besitzen aber keine Stoffwechselaktivität mehr und würden vom Fachmann der Mikrobiologie als tot bezeichnet werden. Somit sind die Milchsäurebakterien nicht mehr befähigt zu wachsen, sich zu teilen, zu replizieren oder Stoffwechselprodukte auszusondern, womit ihr Anwendungsbereich erheblich vergrößert wird.

Die Begriffe "abgetötete" oder "tote Form" und "Derivate" oder "Analoga" oder "Mutanten" schließen vorliegend auch Lysate, Fraktionen oder Extrakte der erfindungsgemäßen Milchsäurebakterien mit ein, wobei diese Lysate, Fraktionen oder Extrakte bevorzugt die Eigenschaften der Milchsäurebakterien besitzen. Hierbei bedeutet "Lysat" - wie auch der Begriff "Extrakt" - insbesondere eine Lösung oder Suspension in einem wässrigem Medium der erfindungsgemäßen Zellen des Milchsäurebakteriums und umfasst bspw. Makromoleküle, wie DNA, RNA, Proteine, Peptide, Lipide, Kohlenhydrate u. ä., sowie insbesondere auch Zelltrümmer. Vorzugsweise umfasst das Lysat auch Zellwand oder Zellwandbestandteile. Verfahren zur Herstellung von Lysaten sind dem Fachmann hinreichend bekannt und umfassen z.B. den Einsatz einer "French Press" oder enzymatische Lyse, Kugelmühle mit Glasperlen oder Eisenkugeln. Das Aufbrechen von Zellen kann enzymatisch, physikalisch oder auch chemisch erfolgen. Beispiele für enzymatische Zelllyse können einzelne Enzyme wie auch Enzymcocktails sein, wie Proteasen, Proteinase K, Lipasen, Glykosidasen; Chemische Lysen können bewirkt werden durch Ionophoren, Detergenzien, wie SDS, Säuren oder Basen; Physikalischen Methoden können durch hohe Drücke, wie French Press, Osmolaritäten, Temperaturen oder Wechsel zwischen Hitze und Kälte bewirkt werden. Ferner können natürlich chemischen, physikalischen und enzymatische Methoden kombiniert werden. "Abgetötete Formen" oder "tote Form" und "Derivate" oder "Analoga" oder "Mutanten" der erfindungsgemäßen Milchsäurebakterien weisen vorzugsweise die gleichen Eigenschaften, wie die benannten Stämme auf. Dabei ist eine metabolische Aktivität bei der "abgetöteten Form" oder" "toten Form" und "Derivate" oder "Analoga" vorzugsweise nicht mehr gegeben. Analoga der erfindungsgemäßen Milchsäurebakterien stellen eine Form des Lysates oder Fragmente dar. Ein Fragment der erfindungsgemäßen Milchsäurebakteriums stellt einen Teil der Zellen dar, wie z.B. Zellmembran, Makromoleküle, wie DNA, RNA, Proteine, Peptide, Lipide, Kohlenhydrate u.ä., sowie auch Zelltrümmer. Mutanten bzw. genetisch veränderte Varianten oder Derivate werden genetisch verändert werden, beispielsweise durch rekombinante DNA-Technologien (Klonen, Sequenzieren, Transformieren rekombinanter Nukleinsäuren), wie auch physikalische Mutagenesen, beispielsweise durch ultraviolette Strahlung, aber auch durch chemische Agenzien , wie mit Ethyl-Methansulfonat (EMS). Dabei können Veränderungen in den positiven Eigenschaften selektiert werden. Genetisch veränderte Mutanten beinhalten Zellen der erfindungsgemäßen Milchsäurebakterien und beherbergen rekombinate Nukleinsäuren in ihrem bakteriellen Chromosom und/oder Plasmiden. Modifikationen durch Punktmutationen können zudem Auswirkungen auf die Expression/Transkription/Translation bewirken, wie auch spontane Mutationen ohne direkte genetische Manipulation. Analoga oder Fragmente können thermisch abgetötete (tote) oder lyophilisierte Formen der erfindungsgemäßen Milchsäurebakterien darstellen, welche ihre erfindungsgemäßen Eigenschaften beibehalten oder verbessern, durch beispielsweise Vergrößerung der Oberfläche. Zellen nach einer Lyophilisation (Gefriertrocknung) sind unter Umständen noch lebensfähig. Diese Zellen können durch spezielle Lagerungsprozesse bei unterschiedlichen Temperaturen abgetötet werden. Tote Zellen können z.B. intakte oder aufgebrochene Zellmembranen besitzen, aber keine metabolische Aktivität. Methoden zur Gewinnung von abgetöteten Zellen können sein, z.B. durch eine Behandlung mit Glasperlen, wobei die Einwirkungen der Scherkräfte zwischen Zellen und Glasperlen zum Aufbruch der Zelle führt. Weitere physikalische Methoden, wie French Press, Hochdruckhomogenisation, Kugelmühle oder aber Einfrier-Tau-Prozesse und Autoklavieren führen zur Abtötung, wie auch zu Fragmenten der erfindungsgemäßen Milchsäurebakterien, wie auch UV-Bestrahlung, Autolyseverfahren oder spezielle Lagerungsprozesse bei unterschiedlichen Temperaturen.

Ob ein Milchsäurebakterium die erfindungsgemäßen Eigenschaften aufweist, kann vom Fachmann bspw. anhand der vorliegend und nachstehend noch beschriebenen Versuche getestet und überprüft werden. Der Begriff "Lactobacillus- Zellen" kann im Sinne der Erfindung auch als Milchsäurebakterien oder Lactobazillen bezeichnet werden und, umfasst solche Mikroorganismen, die Kohlenhydrate, insbesondere Glukose und Lactose, zur Milchsäurevergärung benötigen und zumeist den Embden-Meyerhof-Biosyntheseweg nutzen. Die Lactobacillus Zellen werden taxonomisch in der Familie Lactobacteriaceae zusammengefasst. Sie sind gram-positiv, nicht sporenbildend und im Allgemeinen unbeweglich. Die Lactobacillus Zellen leben anaerob, sind jedoch aerotolerant, obwohl sie keine Hämine (Cytochrom, Katalase) enthalten (Schleifer et al., System. Appl. Microb.: 18, 461-467 (1995) oder Ludwiq et al., System. Appl. Microb. 15: 487-501 (1992). Die Lactobacillus-Zellen bzw. die Spezies können auf der Grundlage des Kohlenhydratverwertungsmusters bestimmt werden, insbesondere mittels dem API Test (Firma biomerieux). Erfindungsgemäß umfasst sind insbesondere solche Spezies, die für eine homofermentative Milchsäuregärung oder heterofermentative Milchsäuregärung geeignet sind. Weiterhin bevorzugt sind solche Lactobacillus- Zellen ausgewählt aus der Gruppe umfassend *Lactobacillus lactis, Lactobacillus helveticus, Lactobacillus jensenii, Lactobacillus acidophilus, Lactobacillus bulgaricus, Lactobacillus amylovorus, Lactobacillus delbrueckii, Lactobacillus casei, Lactobacillus crispatus, Lactobacillus gasseri, Lactobacillus johnsonii, Lactobacillus paracasei, Lactobacillus pentosus, Lactobacillus rhamnosus, Lactobacillus curvatus und Lactobacillus plantarum (alle homofermentativ), weiterhin Lactobacillus brevis, Lactobacillus buchneri, Lactobacillus fructivorans, Lactobacillus hilgardii, Lactobacillus fermentum, Lactobacillus reuteri, Lactobacillus viridescens als auch Bifidobacterium bifidum, Lactobacillus ingluviei* (alle heterofermentativ). Beispielhafte geeignete Lactobacillus Zellen der Anmelderin sind hinterlegt: *Lactobacillus paracasei subsp. paracasei* (DSM 25972), *Lactobacillus crispatus* (DSM 25987, DSM 25988), *Lactobacillus plantarum* (DSM 25989), *Lactobacillus ingluviei* (DSM 25973), sind erfindungsgemäß bevorzugt.

In einer bevorzugten Ausführungsform der vorliegenden Erfindung ist das erfindungsgemäße Milchsäurebakterium ausgewählt aus *Lactobacillus paracasei subsp. paracasei, Lactobacillus crispatus, Lactobacillus plantarum, Lactobacillus ingluviei,* die jeweils unter den Hinterlegungsnummern DSM 25972, DSM 25987, DSM 25988, DSM 25989, DSM 25973, bei der Deutschen Sammlung für Mikroorganismen und Zellkulturen (DSMZ) in Braun-schweig hinterlegt wurden. Die erwähnten DSMZ-Hinterlegungen wurden gemäß dem Budapester Vertrag über die internationale Anerkennung der Hinterlegung von Mikroorganismen zum Zwecke der Patenthinterlegung vorgenommen.

Wie weiter oben erwähnt, betrifft die vorliegende Erfindung auch Zusammensetzungen, enthaltend bevorzugt lebensfähige Milchsäurebakterien, sowie bevorzugt zumindest einen Träger oder Exzipienten, der ausgewählt ist aus zumindest einem der folgenden: einen kosmetisch verträglichen Träger oder Exzipienten, einem pharmazeutisch verträglichen Träger oder Exzipienten oder einem dermatologisch verträglichen Träger oder Exzipienten.

Dabei wird unter einer "Zusammensetzung" im Sinne der vorliegenden Erfindung insbesondere jede Zusammensetzung verstanden, die zumindest ein erfindungsgemäßes Milchsäurebakterium oder dessen Zelllysat aufweist sowie ggf. weitere Inhaltsstoffe wie Träger oder Hilfsstoffe, oder ggf. noch weitere aktive Inhaltsstoffe und Salze. Unter kosmetisch, pharmazeutisch oder dermatologisch verträglichen Hilfsstoffen, Trägern oder Exzipienten wird dabei jede Substanz oder Stoff verstanden, der jeweils üblicherweise im kosmetischen, pharmazeutischen oder im Dentalbereich eingesetzt wird, um einen aktiven Inhaltsstoff der Zusammensetzung, vorliegend also das zumindest eine Milchsäurebakterium oder dessen Zelllysat jeweils kosmetisch oder pharmazeutisch einzusetzen, zu verabreichen und zur Wirkung zu bringen.

Unter "Schleimhaut" im Sinne der vorliegenden Beschreibung und der Ansprüche wird die das Innere von Hohlorganen auskleidende Schicht verstanden, die durch Drüsensekrete feucht gehalten wird. Insbesondere sind damit die Mund- und Nasenschleimhaut, die Bindehaut, die Schleimhaut des Magen-Darm-Traktes und die Schleimhaut des Genitalbereichs gemeint.

Unter "entzündlichen Erkrankungen" werden im Rahmen der vorliegenden Beschreibung und in den Patentansprüchen Erkrankungen verstanden, bei denen es zu akuten, subakuten, chronisch rezidivierenden oder chronisch persistierenden Krankheitszuständen an Haut, Schleimhaut oder in der Mundhöhle kommt. Entzündliche Erkrankungen sind klinisch durch Rötung, Schwellung, Schmerzen, Juckreiz, Exsudation, Blasenbildung, Hyperkeratose, Hypersquamation, Erosionen, Ulzera oder andere Substanzdefekte sowie Krusten, Blasenbildung und andere Effloreszenzen gekennzeichnet. Histologisch finden sich Entzündungszellen im Corium und/oder in der Epidermis. Unter "entzündlichen Erkrankungen" oder "entzündlichen Zuständen" im Sinne der vorliegenden Beschreibung und der Patentansprüche werden insbesondere Erkrankungen und Zustände wie beispielsweise (jedoch nicht erschöpfend) Ekzeme (atopisches Ekzem, seborrhoisches Ekzem, allergische oder toxische Kontaktekzeme), Psoriasis, und andere hyperkeratotische Entzündungen, akute und chronische Wunden, prururiginöse Hauterkrankungen sowie seltenere Entzündungen wie Lichen ruber planus, granulomatöse und parapsoriatische Hauterkrankungen sowie die große Gruppe der Autoimmunerkrankungen mit Hautmanifestation verstanden. Unter "Prophylaxe" werden in der vorliegenden Beschreibung und den Ansprüchen alle Arten der Vorbeugung (Prävention) verstanden, und zwar sowohl die vorbeugende Behandlung bei gesunden Anwendern bzw. Patienten, als auch diejenige bei zu mikrobiell bedingten Erkrankung der Haut, der Schleimhäute oder der Mundhöhle neigenden Anwendern bzw. Patienten oder zu entzündlichen Erkrankungen neigenden Anwendern bzw. Patienten (Personen mit sog. "Disposition"). Weiter wird unter Prophylaxe auch die vorbeugende Behandlung bei Anwendern bzw. Patienten, die bereits einmal unter einer mikrobiell bedingten Erkrankung der Haut, der Schleimhäute oder der Mundhöhle oder unter einer entzündlichen Erkrankung litten und inzwischen die Erkrankung überwunden haben, beispielsweise durch eine erfolgreiche Behandlung wie die in der vorliegenden Beschreibung und den Ansprüchen dargestellte Behandlung (sog. "Rezidivprophylaxe"). Ferner werden unter Prophylaxe im Sinne der vorliegenden Beschreibung und der Ansprüche auch die kosmetische Behandlung sowie die pflegende und reparative Behandlung der zu Irritationen neigenden Haut verstanden.

Es ist bevorzugt, dass die Zusammensetzung nicht nur eines der erfindungsgemäßen Milchsäurebakterien oder deren Zelllysat aufweisen kann, sondern auch eine Mischung von erfindungsgemäßen Milchsäurebakterien oder deren Zelllysat.

Die Zusammensetzung kann bevorzugt in fester oder flüssiger oder viskoser Form oder als Aerosol vorliegen, und kann u.a. in Form von Pulvern, Tabletten, Lösungen, Granulaten, Suspensionen, Emulsionen, Kapseln, Pasten, Gelen, Sprays, etc. vorliegenverwendet werden, mithin in jeder Form, die für eine Verabreichung geeignet ist. Ferner ist bevorzugt, wenn dass die Zusammensetzung weitere Probiotika, Antiseptika oder andere antibakteriell wirksame Substanzen umfasst, sowie bevorzugt ggf. Saccharide und Konservierungsmittel, Geschmacksstoffe, Süßstoffe, Vitamine, Mineralien, Aromen, etc.. Die EP 2 133 414 A1 listet eine Reihe von Inhaltsstoffen, die für bevorzugte Zusammensetzungen eingesetzt werden können. Auf diese Veröffentlichung wird hiermit explizit Bezug genommen. Außerdem können Füllstoffe, Fließmittel, Rheologiemodifikatoren, Weichmacher, Stabilisatoren oder reaktiv vernetzte Monomere, wie z. B. Methacylate in einer bevorzugten Zusammensetzung vorhanden sein. Diesbezüglich kann es bevorzugt sein, dass die Zusammensetzung Substanzen wie EDTA, Magnesium, Calcium, SDS oder deren oder ähnliche Salze enthält. Es war völlig überraschend, dass diese Substanzen zum einen die Biofilmbildung durch *Streptococcus pyogenes* hemmen, aber auch zum anderen die Koaggregationsfähigkeit der bevorzugten Milchsäurebakterien fördern. Die erfindungsgemäße Zusammensetzung wird dabei insbesondere zur Verwendung in der Körperhygiene, -therapie und -prophylaxe eingesetzt, und weist zumindest ein erfindungsgemäßes Milchsäurebakterium oder dessen Zelllysat auf.

Insbesondere ist es bevorzugt, die Zusammensetzung als Probiotikum zu verwenden, also als eine Zubereitung, die lebensfähige Mikroorganismen enthält und die oral eingenommen eine gesundheitsfördernden Einfluss auf denjenigen haben, der das Probiotikum einnimmt bzw. dem dieses verabreicht wird. Derartige Zubereitungen können Nahrungsmittel oder Nahrungsmittelzusätze oder Futtermittel oder Futtermittelzusätze oder aber Medikamente sein. Erfindungsgemäß kann die Zusammensetzung bei allen Nutz- und Haustieren eingesetzt werden, also außer beim Menschen auch bei Hund, Katze, Pferd, Kamel, Falke u.a. Die Milchsäurebakterien bzw. eine diese enthaltene Zusammensetzung kann hierzu unterschiedlich eingesetzt werden. Es kann z. B. bevorzugt sein, sie auf oder in Kauknochen, Kausticks, Tiersnacks, Tierfutter, Pellets, Beiß- und Nagekörper oder Tierspielzeug, usw. gebracht bzw. angewendet werden. Insbesondere ist bevorzugt, wenn die Milchsäurebakterien oder die Zusammensetzung zur Therapie und/oder Prophylaxe von bakterieller Infektionen im Rachenbereich, die insbesondere durch *Streptococcus pyogenes* ausgelöst sind, in Tieren und dem Menschen verwendet wird.

Die Dosierung und die Verabreichung an sich, mit welcher die erfindungsgemäße Zusammensetzung eingesetzt wird, wird vom jeweiligen Einsatz und vom jeweiligen Patienten - insbesondere von dessen Alter, Gewicht, dem allgemeinen Zustand, etc. - abhängen und liegt im Können und der Einschätzung des Fachmanns, der die Zusammensetzung zum Einsatz bringt.

Dabei kann die erfindungsgemäße Zusammensetzung kosmetisch, als Medizinprodukt vorliegen oder pharmazeutisch sein. Vorzugsweise umfasst die Zusammensetzung die Milchsäurebakterien in einer Menge mit einem Masseanteil von 0,001 Gewichtsprozent bis 20 Gewichtsprozent, bevorzugt bis 0,005 Gewichtsprozent bis 10 Gewichtsprozent, besonders bevorzugt 0,01 Gewichtsprozent bis 5 Gewichtsprozent vorliegt. Es war völlig überraschend, dass die Verwendung eines Masseanteils von 0,001 Gewichtsprozent bis 20 Gewichtsprozent insbesondere dazu führte, dass die Zusammensetzung länger nutzbar, d. h. haltbar war. Wenn die Milchsäurebakterien in einer Menge mit einem Masseanteil von 0,005 Gewichtsprozent bis 10 Gewichtsprozent verwendet wurden, führte dies überraschenderweise dazu, dass sich dies positiv auf die rheologischen Eigenschaften der Zusammensetzung auswirkten und diese z. B. eine geringere Viskosität aufweist und somit besser auf der Haut zu verteilen ist oder sich einfacher in den Mundraum einbringen lässt. Eine Verwendung der Milchsäurebakterien in einer Menge mit einem Masseanteil von 0,01 Gewichtsprozent bis 5 Gewichtsprozent hat überraschenderweise dazu geführt, dass sich die Komponenten der Zusammensetzung besser miteinander verbinden und in einer verringerten Arbeitszeit eine homogene Zusammensetzung bereitgestellt werden kann, was wiederum zur Reduzierung der Herstellungskosten führt. Es versteht sich, dass für spezifische Anwendungen aber auch andere Mengen eingesetzt werden können, die sich von den hierin angegebenen unterscheiden. Es war besonders überraschend, dass die erfindungsgemäßen Milchsäurebakterien eine extrem hohe Co-Aggregationaktivität in menschlichem Speichel aufweisen. Die ausgebildeten Aggregate mit *Streptococcus pyogenes* sind außerordentlich stabil und lassen sich dadurch sehr gut und effizient durch Spülen oder Schlucken entfernen.

Überraschenderweise kann die Co-Aggregationseffizienz in Anwesenheit von Co-Faktoren (EDTA, MgCl2, CaCl2, SDS) deutlich gesteigert werden. Es war absolut unerwartet, dass insbesondere EDTA einen ausgesprochenen positiven Effekt auf die Co-Aggregation von erfindungsgemäßen Milchsäurebakterien mit *Streptococcus pyogenes* ausübt. Wie weiter oben erwähnt, kann gemäß einer bevorzugten Ausführungsform die Zusammensetzung bzw. das Milchsäurebakterium im Bereich der Prophylaxe und Behandlung von Hals- und Mandelentzündungen eingesetzt werden, um den genannten pathogenen Keim zu aggregieren, d.h. zu binden. Diese Koaggregate können dann leicht durch Schlucken oder Spülen entfernt werden, bspw. bei einer Suspension insbesondere ausgespült werden, wodurch vorteilhafterweise eine Reduktion des pathogenen Keimes erzielt wird. Die Milchsäurebakterien bzw. eine diese enthaltende Zusammensetzung kann hierzu unterschiedlich eingesetzt werden. Bspw. können sie in Sprays, Gurgel- oder Spüllösungen, als Lutsch- oder Halstabletten, Pastillen, Dragees, Aerosolen, Zahncremes, Säften, Sirup oder als Zusatz in Lebensmitteln bzw. als Nahrungsergänzungsmittel angewendet werden. Daher betrifft die vorliegende Erfindung auch sämtliche Produkte, die im Bereich der Körperhygiene und Medizinprodukte und Prophylaxe eingesetzt werden und die die erfindungsgemäßen Milchsäurebakterien enthalten. Oben beschriebene Ausführungsformen gelten entsprechend für den Bereich der Behandlung, Therapie und Prophylaxe bei Säugetieren. Die Milchsäurebakterien bzw. eine diese enthaltende Zusammensetzung kann hierzu unterschiedlich eingesetzt werden.

Insbesondere ist bevorzugt, wenn die erfindungsgemäße Zusammensetzung und/oder das erfindungsgemäße Milchsäurebakterium zur Herstellung eines Arzneimittels, zur Behandlung oder Prophylaxe von Hals- und Rachenbeschwerden, *Pharyngitis atrophicans et sicca, Pharyngitis sicca, Angina lateralis,* Tonsillitis durch *Streptococcus pyogenes* verwendet wird. Durch die erfindungsgemäßen Milchsäurebakterien und die diese enthaltende Zusammensetzung werden Mittel bereit gestellt, mit welchen diese Erkrankungen vorteilhaft behandelt, bzw. vermieden werden können. Die Milchsäurebakterien bzw. die diese enthaltenden Zusammensetzungen können dabei sowohl in der Human- als auch in der Veterinärmedizin, insbesondere, wie bereits aufgeführt, bei Hund, Katze, Pferd, Kamel, Falke eingesetzt werden. Die erfindungsgemäße Zusammensetzung bzw. die Milchsäurebakterien können insbesondere als Nahrungsmittelzusatz, als Hygieneprodukt, bzw. als ein die Milchsäurebakterien aufweisendes Hygieneprodukt, oder als eine pharmazeutische Präparation eingesetzt werden.

Die vorliegende Erfindung betrifft ferner ein Verfahren zur Identifizierung und/oder Selektion eines Mikroorganismus der Gattung Lactobacillus sp. mit der/den erfindungsgemäßen Eigenschaft/en, wobei das Verfahren zumindest die folgenden Schritte aufweist: a) Inkubieren eines Ansatzes aus einem pathogenen Mikroorganismus, ausgewählt aus *Streptococcus pyogenes,* zur Bindung an Fibronectin, b) Zugabe des zu untersuchenden Mikroorganismus der Gattung Lactobacillus und Inkubation des Ansatzes zur Ausbildung der spezifischen Bindung zwischen dem pathogenen Mikroorganismus und dem zu untersuchendem Mikroorganismus der Gattung Lactobacillus c) Abtrennen der nicht gebundenen Mikroorganismen der Gattung Lactobacillus durch Entfernen des Überstandes und d) Bestimmung der Fibronectinbindung im Hinblick auf gebundene und aggregierte Mikroorganismen der Gattung Lactobacillus.

In einer bevorzugten Ausführungsform weist das erfindungsgemäße Verfahren noch den Schritt der Untersuchung zur Verhinderung der Fibronectinbindung durch *Streptococcus pyogenes* auf. Die Zugabe der zu untersuchenden Mikroorganismen der Gattung Lactobacillus erfolgt dabei während der Inkubation des Fibronectinbindenden fluoreszenzmarkierten pathogenen Mikroorganismus. Nach Entfernung der nicht gebundenen Zellen erfolgt bevorzugt eine spezifische Quantifizierung der Bindung an Fibronectin durch Messung der Fluoreszenz im Vergleich zu einer Kontrolle ohne Zugabe der zu testenden Mikroorganismen.

Es versteht sich, dass die vorstehend genannten und nachstehend noch zu erläuternden Merkmale nicht nur in der jeweils angegebenen Kombination, sondern auch in Alleinstellung verwendbar sind, ohne den Rahmen der vorliegenden Erfindung zu verlassen.

### BESCHREIBUNG DER FIGUREN

Weitere Vorteile ergeben sich aus den nachstehenden Figuren und den hierzu durchgeführten Versuchen und Ausführungsbeispielen. Die Experimente wurden mit allen bevorzugten Milchsäurebakterien durchgeführt, wobei nur ein Teil der Experimente dargestellt ist. Der Fachmann kann anhand der Beispiele die Erfindung nacharbeiten. Es zeigen:

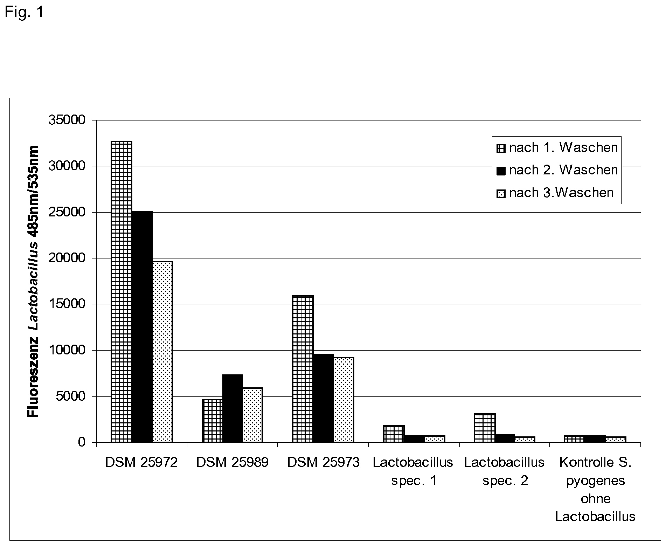

Fig. 1 zeigt die spezifische Bindung bzw. Aggregation eines Ausführungsbeispiels der erfindungsgemäßen Mikroorganismen (DSM 25972, DSM 25989, DSM 25973 an durch *Streptococcus pyogenes* ausgebildeten Biofilm (Bindungs-Assay) nach 3 maligen Auswaschen der nicht-gebundenen Zellen sowie im Vergleich dazu weitere, nicht erfindungsgemäße *Lactobacillus* Stämme, die nicht zur Bindung an *Streptococcus pyogenes* befähigt sind (*Lactobacillus* spec.1 und *Lactobacillus* spec.2); spezifische Quantifizierung der Bindung bzw. Aggregation von CFDA-markierten erfindungsgemäßen *Lactobacillus* Stämmen an den S. *pyogenes* Biofilm in 96-Well-Mikrotiterplatte über Fluoreszenzmessung (Ex485nm/Em535nm). Hierdurch erfährt der Fachmann, dass die bevorzugten Mikroorganismen insbesondere bei bakteriellen Infektionen verwendbar sind, die insbesondere im Mund- oder Rachenraum erfolgen.

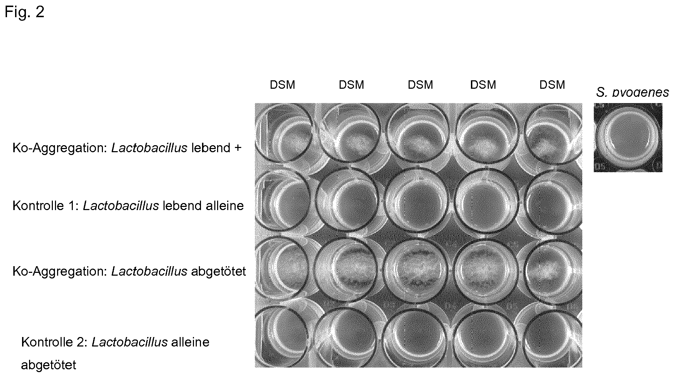

Fig. 2 stellt die makroskopische Kontrolle (Foto einer 24 well Mikrotiterplatte) der spezifischen Bindung eines Ausführungsbeispiels eines erfindungsgemäßen Mikroorganismus (DSM 25972, DSM 25987, DSM 25988, DSM 25989 und DSM 25973 an *Streptococcus pyogenes* im Ko-Aggregationsansatz im 24-Well-Maßstab dar sowie die erfindungsgemäßen *Lactobacillus* Stämme sowie der Targetstamm *Streptococcus pyogenes* alleine in einer Photodokumentationsanlage. Weißer Niederschlag wird durch Ko-Aggregation (und eine damit verbundene Ausfällung) verursacht. Die Agglomerate führen dazu, dass *Streptococcus pyogenes* insbesondere nicht an die Epithelzellen des Rachens binden kann. Versuche bei Patienten haben gezeigt, dass mittels den bevorzugten Mikroorganismen bakterielle Infektionen verhindert, bzw. reduziert werden können.

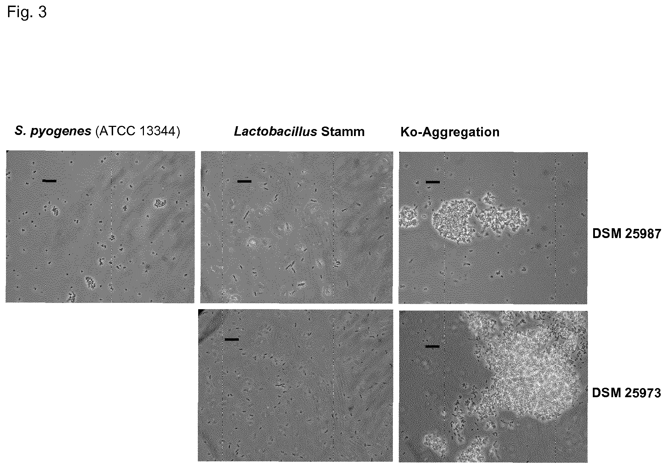

Fig. 3 zeigt die mikroskopische Darstellung der spezifischen Bindung eines Ausführungsbeispiels eines erfindungsgemäßen Mikroorganismus (DSM 25987 und DMS 25973) an *Streptococcus pyogenes* Zellen nach der Koaggregation, sowie die erfindungsgemäßen *Lactobacillus* Stämme und der *Streptococcus pyogenes* alleine. Mikroskopische Abbildung (Phasenkontrast, 1000x vergrößert). Koaggregate sind als große Zellagglomerationen zu sehen.

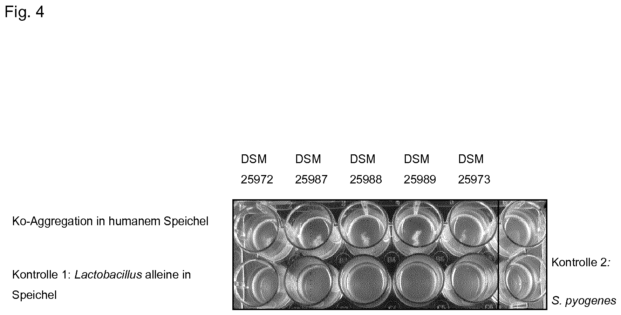

Fig. 4 zeigt die makroskopische Kontrolle (Foto einer 24 well Mikrotiterplatte) der spezifischen Bindung eines Ausführungsbeispiels eines erfindungsgemäßen Mikroorganismus (DSM 25972, DSM 25987, DSM 25988, DMS 25989 und DMS 25973) an *Streptococcus pyogenes* Zellen (Koaggregation) in humanem Speichel sowie die erfindungsgemäßen *Lactobacillus* Stämme und der *Streptococcus pyogenes* alleine in einer Photodokumentationsanlage. Weißer Niederschlag wird durch Koaggregation (und eine damit verbundene Ausfällung) verursacht. Es war überraschend, dass die bevorzugten Mikroorganismen bereits im Speichel an *Streptococcus pyogenes* binden, insbesondere koaggregieren und so eine Bindung von *Streptococcus pyogenes* an Epithelzellen verhindern. Es war zudem überraschend, dass die bevorzugten Mikroorganismen ihre bevorzugten Fähigkeiten nicht durch die im Speichel enthaltenen Enzyme oder Bestandteile verlieren. Hierdurch sind die bevorzugten Mikroorganismen vorteilhaft für die Verwendung in einem oralen Applikationsmittels, wie insbesondere einem Bonbon.

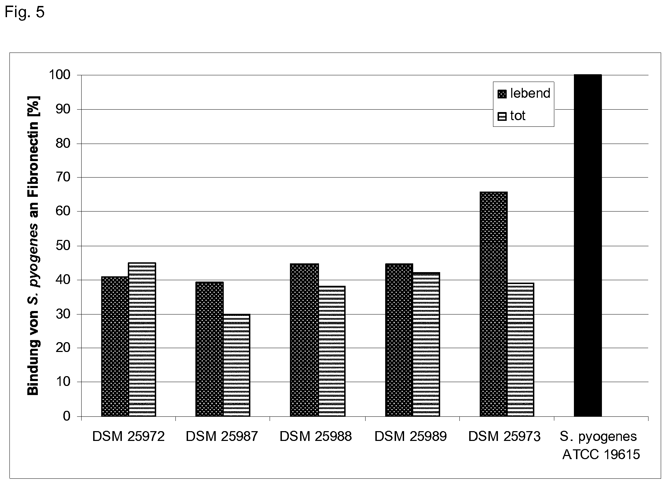

Fig. 5 zeigt den Einfluss eines Ausführungsbeispiels der erfindungsgemäßen Mikroorganismen (DSM 25972, DSM 25987, DSM 25988, DSM 25989 und DSM 25973) auf die Verhinderung der Fibronectinbindung von *Streptococcus pyogenes* Stamm ATCC 19615 nach 2 h Inkubation bei 37 °C mit in PBS gewaschener Lactobacillus -Suspension (lebend) und durch Hitzebehandlung abgetötete Lactobacillus-Suspension (tot). Spezifische Quantifizierung der Bindung von CFDA-markierten *Streptococcus pyogenes* an die mit Fibronectin beschichte 96-Well-Mikrotiterplatte. Dargestellt ist die Bindung von *Streptococcus pyogenes* in %. 100% repräsentiert die Bindung des S. *pyogenes* (ATCC 19615) alleine an die Fibronectinbeschichtung der Mikrotiterplatte ohne Zugabe von *Lactobacillus* -Suspension. Es zeigt sich, dass die bevorzugten Mikroorganismen die Bindung von *Streptococcus pyogenes* an Epithelzellen im Rachen verhindern und somit bakteriellen Infektionen vorbeugen.

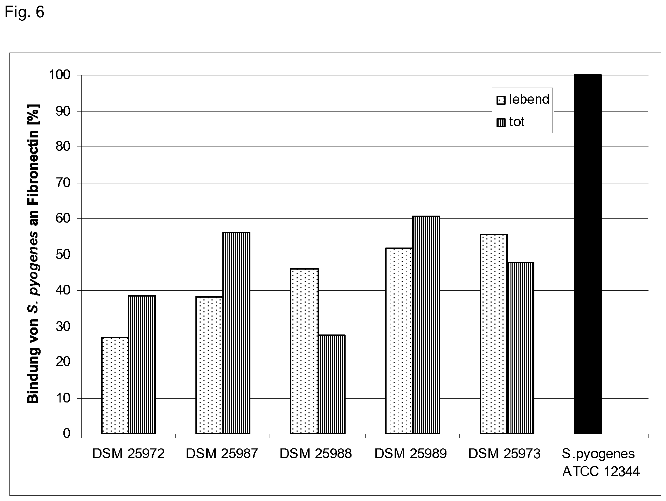

Fig. 6 zeigt den Einfluss eines Ausführungsbeispiels der erfindungsgemäßen Mikroorganismen (DSM 25972, DSM 25987, DSM 25988, DSM 25989 und DSM 25973 auf die Bindung von *Streptococcus pyogenes* (Stamm ATCC 12344) an Fibronectin nach 2 h Inkubation bei 37 °C (mit in PBS gewaschener Lactobacillus-Suspension (lebend) und durch Hitzebehandlung abgetötete Lactobacillus-Suspension (tot)). Spezifische Quantifizierung der Bindung von CFDA-markierten *Streptococcus pyogenes* an die mit Fibronectin beschichte 96-Well-Mikrotiterplatte. Dargestellt ist die Bindung von *Streptococcus pyogenes* in %. 100% repräsentiert die Bindung des *S*. *pyogenes* alleine an die Fibronectinbeschichtung der Mikrotiterplatte ohne Zugabe von *Lactobacillus* -Suspension.

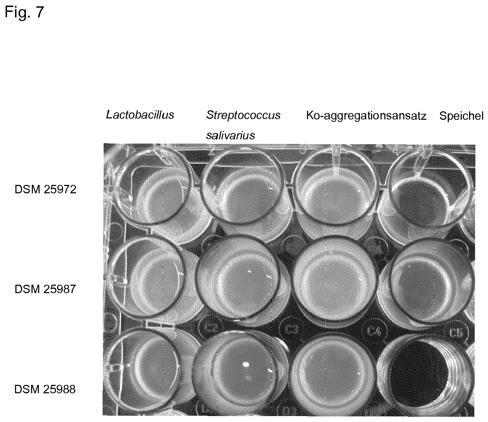

Fig. 7 zeigt die makroskopische Darstellung (Foto einer 24 well Mikrotiterplatte) der Koaggregation eines Ausführungsbeispiels der erfindungsgemäßen Mikroorganismen (DSM 25972, DSM 25987, DSM 25988, DMS 25989 und DMS 25973) und *Streptococcus salivarius* in PBS Puffer sowie die erfindungsgemäßen *Lactobacillus* Stämme und *Streptococcus salivarius* alleine in einer Photodokumentationsanlage. Weißer Niederschlag wird durch Koaggregation (und eine damit verbundene Ausfällung) verursacht.

### DETAILLIERTE BESCHREIBUNG DER AUSFÜHRUNGSBESPIELE

### Beispiel 1:

Zur Identifizierung und Selektion von erfindungsgemäßen Mikroorganismen wurden aus einer Lactobacillus-Stammbank verschiedene Stämme durch ein vier-stufiges Screening untersucht, wobei zunächst hinsichtlich der Bindungsfähigkeit an pathogenen Targetkeim *Streptococcus pyogenes* (im Folgenden auch "Targetkeim") gescreent wurde (Bindungs-Assay), und anschließend die im ersten Schritt identifizierten Stämme in einem Ko-Aggregationsassay im Mikrotiterplatten-Maßstab geprüft wurden, wobei die Ko-Aggregation mit dem jeweiligen Targetkeim qualitativ mittels binokularen Stereomikroskops nachgewiesen wurde. Der Begriff "Ko-Aggregation" wird im Sinne der Erfindung synonym zu dem Begriff "Koaggregation" verwendet. Ferner wurde die Stärke der Ko-Aggregation und die Stabilität der Bindung mit dem Targetkeim, sowie die Fähigkeit zur Verhinderung der Bindung an Fibronectin untersucht, was schließlich zu den identifizierten beispielhaften erfindungsgemäßen Mikroorganismen (Lactobacillus) führte.

### Bindunas-Assay

Um die Bindungsaktivität selektierter *Lactobacillus* Stämme quantifizieren zu können, wurde ein *Bindungs-Assay* etabliert, welcher den quantitativen Nachweis der Bindung der *Lactobacillus* Stämme an pathogenen Mikroorganismus in einer 96-Well-Platte ermöglichte. Die Bindungsaktivität der *Lactobacillus* Zellen mit dem Targetkeim korreliert mit der Ko-Aggregationsaktivität bzw. Ko-Aggregationsfähigkeit. Dies wurde experimentell überprüft. Dazu zeigt Fig. 2 exemplarisch die Ko-Aggregation des *Lactobacillus* DSM 25972, DSM 25987, DSM 25988, DSM 25989 und DSM 25973 und *Streptococcus pyogenes* ATCC 19615.

Die Messmethode basiert auf der spezifischen Bindung der erfindungsgemäßen Stämme an in einem Biofilm gebundenen Targetstamm. Für den Assay werden definierte Mengen der erfindungsgemäßen Stämme mit einem Fluoreszenzfarbstoff (CFDA-Lösung, Invitrogen) markiert und mit einer definierten Menge an in einem Biofilm gebundenen Targetkeimen gemischt. Der Verbleib der gebundenen, erfindungsgemäßen Stämme an dem Targetstamm wird nach mehrmaligem Waschen mittels Fluoreszenzphotometer vermessen.

Zur Durchführung dieser Versuche wurde der Targetkeim *Streptococcus pyogenes* nach dem Standardprotokoll kultiviert. Die Kultivierung des Stammes erfolgte in THY (Todd Hewitt Broth) -Medium unter aeroben Bedingungen, in 5% CO₂-Atmosphäre, bei 37°C. Die *Lacto*bacillus-Stämme wurden in MRS-Medium (siehe de Man et al., (1960) "A medium for the Cultivation of Lactobacilli", J. Appl. Bact. 23(130-135) anaerob bei 37 °C kultiviert. Zur Aufarbeitung des Targetkeimes wurden die Zellen in der mittleren exponentiellen Wachstumsphase nach 5-6 h Kultivierung geerntet, 3 Mal mit PBS (Phosphat-gepufferter Saline, pH 7,4) gewaschen und in PBS aufgenommen. Dann wurden 100 µl der Suspension pro Well in eine 96-Well-Mikrotiterplatte vorgelegt. Während einer 16stündigen Inkubation bei 37°C in 5% CO₂ kommt es zur Ausbildung eines Biofilmes durch den Targetstamm. Nach der Inkubation werden nicht gebundene Zellen durch 3-maliges Waschen mit Phosphat-gepufferter Saline (PBS, pH 7,4) entfernt. Zur Aufarbeitung der *Lactobacillus-Stämme* wurden diese nach 24stündiger Kultivierung geerntet, 3 Mal mit PBS gewaschen, in PBS aufgenommen und durch Zugabe der CFDA-Lösung (Invitrogen) fluoreszenzmarkiert. Zur Durchführung des Bindungs-Assays wurden zu den in einem Biofilm gebundenen Targetstamm pro Well 100µl der fluoreszenzmarkierten Lactobacillus-Suspension hinzugefügt. Parallel erfolgten Kontrollansätze ohne Zugabe der *Lactobacillen-Suspension.* Nach 1 stündiger Inkubation bei 37 °C in einem Brutschrank wurden die nicht gebundenen Zellen abgetrennt und 3 Mal mit PBS gewaschen. Nach jedem Waschschritt wurde die Fluoreszenz im Fluoreszenzplattenphotometer (Em485nm/Ex535nm) gemessen. Die Zunahme der Fluoreszenz (Em 485 nm/Ex 535 nm) im Ansatz mit *Lactobacillus* Stämmen im Vergleich zur Kontrolle ohne *Lactobacillus* Zellen korrelierte dabei mit der Menge an Biofilm-gebundenen *Lactobacillus* Zellen, Die gemessene Fluoreszenz nach Bindung der *Lactobacillus* Zellen entsprach der Bindungsstärke. Je grösser dieser Wert war, desto besser haben die markierten *Lactobacillus* Zellen an die im Biofilm gebundenen Targetstämme gebunden und desto stärker ist die Bindungsaktivität der untersuchten *Lactobacillus-Zellen.*

Die Versuche ergaben, dass die erfindungsgemäßen *Lactobacillus* Stämme für den Targetkeim *Streptococcus pyogenes* im *Bindungs-Assay* nach Entfernung der ungebundenen Zellen zu einer bis 17-fachen Erhöhung der Fluoreszenz infolge der Bindung der markierten *Lactobacillus* Zellen an die im Biofilm gebundenen Targetstämme nach 3 Waschungen führte. Beispielhaft für die erfindungsgemäßen Mikroorganismen sind in Figur 1 die Ergebnisse des Bindungs-Assays für *Lactobacillus* DSM 25972, DSM 25989 und DSM 25973 mit *Streptococcus pyogenes* dargestellt. Weiterhin sind in Figur 1 zum Vergleich die Daten nicht erfindungsgemäßer *Lactobacillus* Stämme dargestellt (*Lactobacillus* spec. 1 und 2), welche nicht zur spezifischen Bindung an den Targetkeim befähigt sind. Es zeigt sich, dass die bevorzugten Mikroorganismen bakterielle Infektionen bei Mensch und Tier verhindern können, bzw. zur Behandlung dieser eingesetzt werden, da sie spezifisch mit *Streptococcus pyogenes* koaggregieren. Hierdurch wird zum einen eine Bindung von *Streptococcus pyogenes* an Epithelzellen verhindert. Zum anderen wird die Biofilmbilsung durch *Streptococcus pyogenes* verhindert, bzw. bereits gebildete Biofilme aufgelöst.

### Beispiel 2:

### Ko-Aggregationsassay

Um die Co-Aggregationsaktivität selektierter *Lactobacillus* Stämme zu veranschaulichen, dient der nachfolgende Nachweis im 1,0 ml-Volumen bzw. in einer 24-Well-Platte.

Bei dieser Methode wird die Aggregationsverhalten der Lactobacillen, sowohl des Targetkeims separat betrachtet und schließlich die Ko-Aggregation, *Lactobacillus* und Targetstamm zusammen im Gemisch. Die Betrachtung erfolgt makroskopisch durch Fotographie der 24-Well-Platte, wie auch mikroskopisch. Zur Durchführung dieser Versuche wurde der Targetkeim *Streptococcus pyogenes* nach Standardprotokollen kultiviert. Die Kultivierung des Stammes erfolgte in THY (Todd Hewitt Broth) -Medium unter aeroben Bedingungen, in 5% CO2-Atmosphäre, bei 37°C. Die *Lactobacillus* Stämme wurden in MRS-Medium (siehe de Man et al., (1960) "A medium for the Cultivation of Lactobacilli", J. Appl. Bact. 23(130-135) anaerob bei 37 °C kultiviert. Zur Aufarbeitung des Targetkeimes wurden die Zellen in der mittleren exponentiellen Wachstumsphase nach 5-6 h Kultivierung geerntet, 2 Mal mit Phosphat-gepufferter Saline (PBS, pH 7,4) gewaschen und auf die OD600=4 justiert. Zur Aufarbeitung der Lactobacillus-Stämme wurden diese nach 16h Kultivierung geerntet, 2 Mal mit PBS gewaschen, anschließend in PBS-Volumen aufgenommen und entsprechend auf eine OD600=4 justiert. Zur Durchführung des Ko-Aggregationsassays wurden je 500 µl einer Targetkeim-Suspension pro Well mit 500 µl Lactobacillus-Suspension in einer 24-Well-Platte vermengt. Parallel erfolgten Kontrollansätze mit 500 µl des Targetkeims plus 500 µl PBS, oder 500 µl der jeweiligen Lactobacillus-Suspension plus 500 µl PBS (Kontrolle 1). Nach 10 minütiger Inkubation bei 25 °C auf einem Tischschüttler wurden die Ansätze makroskopisch mittels Photodokumentationsanlage, wie auch mikroskopisch betrachtet. Zur Untersuchung der Ko-Aggregationsfähigkeit der abgetöteten *Lactobacillus* Zellen wurden diese zunächst durch Hitzebehandlung bei 70°C für 30 min im Wasserbad abgetötet und danach in den Ko-Aggregationsassay eingesetzt.

Die Versuche zeigen, dass die selektierten *Lactobacillus* Stämme mit dem Targetkeim *Streptococcus pyogenes* koaggregieren, so dass Verklumpungen (Aggregaten) im Gemisch beobachtet werden konnten, welche makroskopisch durch ungleichmäßige, kondensierte und/oder körnige Bereiche im Well sichtbar sind. In den Wells, welche den Targetstamm bzw. die erfindungsgemäßen *Lactobacillus* Stämme separat enthält kommt es zu keiner Verklumpung bzw. Aggreagatbildung. Die Zellsuspension im Well bleibt homogen. Die makroskopischen Resultate im Ko-Aggregationsansatz sind für die selektierten *Lactobacillus* Stämme sowohl lebend als auch abgetötet für den Targetkeim *Streptococcus pyogens* in Figur 2 dargestellt. In der mikroskopischen Betrachtung zeigen sich durch alle erfindungsgemäßen *Lactobacillus* Stämme eine eindeutige Affinitäten zu dem Targetkeim, welche zu unterschiedlichen Aggegatgrößen in der mikroskopischen Betrachtung der Ko-Aggregation führt. Die mikroskopischen Ergebnisse im Ko-Aggregationsansatz sind beispielhaft für die erfindungsgemäßen *Lactobacillus* Stämme DSM 25987 und DSM 25973 für den Targetkeim *Streptococcus pyogenes* in Figur 3 dargestellt. Insbesondere bei Patienten oder Tieren können die bevorzugten Mikroorganismen bakterielle Infektionen, die durch *Streptococcus pyogenes* ausgelöst sind, bzw. an denen *Streptococcus pyogenes* beteiligt ist, verhindert oder bekämpft werden

### Beispiel 3:

### Ko-Aggregation in humanem Speichel

Zur Untersuchung der Ko-Aggregationsfähigkeit der erfindungsgemäßen Lactobacillus Stämme in natürlichem humanen Speichel, wurden diese nach der 16stündigen Kultivierung , wie im Beispiel 2 beschrieben, geerntet, 2 Mal in PBS gewaschen und in humanem Speichel aufgenommen. Der Speichel wurde vorher von mehreren Personen gesammelt, gemischt und durch Zentrifugation bei 8000g für 20 min von Partikeln getrennt. Der Targetkeim wurde wie im Beispiel 2 beschrieben aufgearbeitet und ebenso nach 2 Mal Waschen in PBS in humanem Speichel aufgenommen. Zur Durchführung des Ko-Aggregationsassays wurden je 500 µl Targetkeim-Suspension im Speichel mit 500 µl Lactobacillus-Suspension im Speichel pro Well in einer 24-Well-Platte vermengt. Parallel erfolgten Kontrollansätze mit 500 µl des Targetkeims plus 500 µl Speichel, oder 500 µl der jeweiligen Lactobacillus-Suspension plus 500 µl Speichel (Kontrolle 1). Nach 10 minütiger Inkubation bei 25 °C auf einem Tischschüttler wurden die Ansätze makroskopisch mittels Photodokumentationsanlage, wie auch mikroskopisch betrachtet.

Die Versuche zeigen, dass die erfindungsgemäßen Lactobacillus Stämme in natürlichem Speichel zur einer spezifischen Ko-Aggregation mit dem Targetkeim Streptococcus pyogenes fähig sind. Das stellt die besten Voraussetzungen zur Applikation der erfindungsgemäßen Lactobacillus Stämme im Mund-Rachenraum dar. Hierfür können die bevorzugten Mikroorganismen zum Beispiel als Bonbon oral verabreicht werden und führen zur Linderung bzw. vollständigen Therapie von bakteriellen Infektionen. Die makroskopischen Ergebnisse der Ko-Aggregation in natürlichem humanem Speichel sind für die erfindungsgemäßen Lactobacillus Stämme mit dem Targetkeim Streptococcus pyogens in Figur 4 dargestellt.

### Beispiel 4:

### Verhinderung der Bindung an Fibronectin

Zur Untersuchung der Wirkung der erfindungsgemäßen *Lactobacillus* Stämme auf die Bindung des pathogenen Targetkeimes *Streptococcus pyogenes* (ATCC 19615) an Fibronectin wurde ein Bindungsassay etabliert, welcher den quantitativen Nachweis der Bindung von fluoreszenzmarkierten Targetkeim in einer mit Fibronectin beschichteten 96-Well-Platte ermöglichte. Die erfindungsgemäßen *Lactobacillus* Stämme wurden direkt zu dem CFDA-markiertenTargetkeim zu Beginn der Bindung zugegeben und für 2 h bei 37°C inkubiert. Nach der Entfernung der nicht gebundenen Zellen und 2 x Waschen mit PBS erfolgte die spezifische Quantifizierung. der gebunden Targetkeimzellen durch Messung der Fluoreszenz bei Em 485nm/Ex 535nm im Fluoreszenzplattenphotometer. Zur Durchführung dieser Versuche wurden Targetstämme und erfindungsgemäßen Lactobacillus-Stämme nach Standard-Protokollen kultiviert. Zur Aufarbeitung der Lactobacillus-Stämme wurden diese nach Kultivierung 2 Mal mit PBS gewaschen und in PBS aufgenommen. Ein Teil der *Lactobacillus* Stämme wurde nach PBS-Waschung mittels Pasteurisation bei 70°C für 30min abgetötet. Zur Aufarbeitung des Targetstammes wurde dieser bis zum Erreichen der stationären Wachstumsphase für 16 h kultiviert, geerntet, durch Zugabe von CFDA fluoreszenzmarkiert und auf die OD600nm =3,0 eingestellt. Zur Durchführung des Bindungs-Assays wurden die *Lactobacillus* Zellen direkt zu dem fluoreszenzmarkierten Targetkeim zu Beginn der Bindung zugegeben und für 2 h, mikroaerophil, bei 37 °C inkubiert. Parallel erfolgten Kontrollansätze ohne Zugabe der *Lactobacillus* Suspension. Nach der Entfernung der planktonischen Zellen und Waschen der im Biofilm gebundenen Zellen erfolgte die spezifische Quantifizierung der gebundenen Targetkeimzellen durch Fluoreszenzmessung mit bzw. ohne *Lactobacillus* Zellen. Die Reduktion der Fluoreszenz im Vergleich zur Kontrolle des Targetkeims ohne Lactobacillen korrelierte dabei mit der Stärke der Bindung des Targetkeimes *Streptococcus pyogenes* an mit Fibronectin beschichtete Mikrotiterplatte. Diese Reduktion wird als prozentuale Verhinderung der Biofilmbildung dargestellt. Beispielhaft für die erfindungsgemäßen Mikroorganismen ist das Ergebnis der Verhinderung der Bindung an Fibronectin durch die *Lactobacillus* Stämme DSM 25972, DSM 25987, DSM 25988, DSM 25989 und DSM 25973 , in Figur 5 exemplarisch an *Streptococcus pyogenes* ATCC 19615 dargestellt. Die Versuche ergaben, dass es zu einer Verhinderung der Bindung an Fibronectin von Targetkeim durch die oben genannten erfindungsgemäßen *Lactobacillus* Stämme sowohl in lebender als auch in abgetöteter Form führt. Somit wurde gezeigt, dass die erfindungsgemäßen *Lactobacillus* Stämme in der Lage sind die Bindung von *Streptococcus pyogenes* an Wirtszellen zu verhindern. Ferner wurden Versuche mit weiteren Keimen der gleichen Gattung und Spezies durchgeführt, um die Spezifität der Bindungs- bzw. Aggregationseigenschaften der erfindungsgemäßen *Lactobacillus* Stämme sowie ihre Fähigkeit zur Verhinderung der Bindung an Fibronectin gegen die Targetkeime der gleichen Gattung und Spezies darzustellen. Dafür wurde unter anderem *Streptococcus pyogenes* ATCC 12344 ausgewählt. Zur Untersuchung ihrer Wirkung auf die Bindung des Targetkeimes *Streptococcus pyogenes* ATCC 12344 an Fibronectin wurden die erfindungsgemäßen *Lactobacillus* Stämme und der Targetkeim wie oben unter Standardbedingungen kultiviert, aufgearbeitet und in den Assay eingesetzt.

Die Versuche ergaben, dass auch weitere Keime der gleichen Art und Gattung durch die erfindungsgemäßen *Lactobacillus* Stämme aggregiert werden können sowie eine Verhinderung der Bindung an Fibronectin weiterer Targetkeime durch die erfindungemäßen *Lactobacillus* Stämme erreicht wird. Beispielhaft für die erfindungsgemäßen Mikroorganismen sind in Figur 6 die Ergebnisse der Verhinderung der Bindung von *Streptococcus pyogenes* (Stamm ATCC 12344) an Fibronectin durch die *Lactobacillus Stämme* DSM 25972, DSM 25987, DSM 25988, DSM 25989 und DSM 25973 dargestellt. Es war völlig überraschend, dass bei Menschen oder Tieren eine Verabreichung von bevorzugten Mikroorganismen zu Verhinderung oder Therapie von bakteriellen Infektionen genutzt werden kann, wobei an der Infektion auch weitere Keime der gleichen Art und Gattung wie *Streptococcus pyogenes* beteiligt sein können.

### Beispiel 5:

### Ko-Aggregation von Streptococcus salivarius

Erfindungsgemäße *Lactobacillus* Stämme und *Streptococcus salivarius* wurden nach Standardbedingungen kultiviert, in PBS 3 Mal gewaschen und im Ko-Aggregationsassay in humanem Speichel eingesetzt. Die Ergebnisse zeigen, dass *Streptococcus salivarius* von erfindungsgemäßen *Lactobacillus* Stämmen nicht koaggregiert wird (Abbildung 7). Demnach kann ausgeschlossen werden, dass Kommensale, hier exemplarisch repräsentiert durch *Streptococcus salivarius,* von erfindungsgemäßen *Lactobacillus* Stämmen nicht koaggregiert und von ihrem natürlichen Habitat entfernt werden. Dies zeigt insbesondere, dass die bevorzugten Milchsäurebakterien spezifisch gegen bakterielle Infektionen wirken.

### REFERENZLISTE

Beachey und Ofek (1976) Epithelial cell binding of Group A Streptococci by lipoteichoic acid on fimbriae denuded of M protein. Journal of experimental medicine 143:759-771
Bisno (1995) Streptococcus pyogenes S. 1786ff in Mandell, Bennett und Dolin (Hrsg.) Principles and practice of infectious diseases. Vol 2. Churchill Livingstone, New York
Courtney et al. (1999) Strategies for prevention of group A streptococcal adhesion and infection. S 553ff. In An und Friedman (Hrsg.) Handbook of bacterial adhesion: principles, methods, and applications. Humana Press, Totowa, N.J
Cunningham (2000) Pathogenesis of group A streptococcal infections. Clin Microbiol Rev 13:470-511
Hasty et al. (1992) Multiple adhesins of streptococci. Infect Immun 60: 2147-2152
Kaplan (1991) The resurgence of group A streptococcal infections and their sequelae. Eur J Clin Microbiol Infect Dis 10:55-57
Kilian (2002) Streptococcus and Enterococcus. In: Medical Microbiology. Greenwood, D.; Slack, RCA.; Peutherer, J.F. (Hrsg.) Kapitel 16. Churchill Livingstone, Edingburgh, UK: pp. 174-188, 2002
LaPenta et al. (1994). Group A streptococci efficiently invade human respiratory epithelial cells. Proc. Natl. Acad. Sci. USA 91:12115-12119
Musser und Krause (1998) The revival of group A streptococcal diseases, with a commentary on staphylococcal toxic shock syndrome. In Emerging Infections. Krause (Hrsg.) Academic Press, New York
Reid et al. (2001) Group A Streptococcus: Allelic variation, population genetics, and host pathogen interactions. J Clinic Invest 107: 393-399
Simpson und Beachey (1983) Adherence of group A streptococci to fibronectin on oral epithelial cells. Infect Immun 39:275-279

## Patentansprüche

1. Milchsäurebakterium oder dessen Zelllysat zur Prophylaxe und/oder Therapie mikrobiell bedingter Erkrankungen der Haut, Schleimhäute und/oder der Mundhöhle, wobei das Milchsäurebakterium mit einem Streptococcus pyogenes koaggregiert, wobei das Milchsäurebakterium ausgewählt ist aus der Gruppe umfassend folgende bei der Deutschen Sammlung für Mikroorganismen und Zellkulturen hinterlegten als DSM 25972, DSM 25987, DSM 25988, DSM 25989 und DSM 25973 nummerierten Mikroorganismen.

2. Milchsäurebakterium, oder dessen Zelllysat, nach Anspruch 1, wobei die Fähigkeit zur Koaggregation des Streptococcus pyogenes auch nach einer biologischen, chemischen oder physikalischen Behandlung besteht und/oder wobei die Fähigkeit zur Koaggregation des Streptococcus pyogenes bei einem pH-Wert von zwischen ca. 3 bis ca. 8 besteht.

3. Milchsäurebakterium, oder dessen Zelllysat nach einem oder mehreren der vorherigen Ansprüche, wobei das Milchsäurebakterium, oder dessen Zelllysat die Fähigkeit zur Hemmung einer Bildung eines Biofilms des Streptococcus pyogenes besitzt und/oder wobei das Milchsäurebakterium oder dessen Zelllysat die Fähigkeit zur Verhinderung der Bindung an Fibronektin durch Streptococcus pyogenes aufweist und/oder wobei das Milchsäurebakterium, oder dessen Zelllysat, die Fähigkeit aufweist, keinen kommensalen Mikroorganismus der Haut oder der Schleimhäute zu koaggregieren.

4. Zusammensetzung umfassend mindestens ein Milchsäurebakterium, oder dessen Zelllysat, nach einem oder mehreren der vorherigen Ansprüche, bevorzugt
wobei die Zusammensetzung einen pharmazeutisch oder kosmetisch verträglichen Träger oder Exzipienten umfasst und/oder
wobei die Zusammensetzung in fester, flüssiger, viskoser Form oder als Aerosol vorliegt und/oder
wobei die Zusammensetzung als Paste, Weichgelatinekapsel, Hartgelatinekapsel, Pulver, Granulat, Kügelchen, Pastille, Brausetablette, Lutschtablette, Buccaltablette, Kautablette, Subligualtablette, Lösung, Tinktur, Emulsion, Saft, Konzentrat, Sirup, Spray, Trinkampulle, Gel, Mundwasser, Zahnpulver, Kaugummi, Tablette, Dragee oder Bonbon vorliegt und/oder
wobei die Zusammensetzung Probiotika, Antiseptika oder andere antibakteriell wirksame Substanzen umfasst und/oder
wobei die Zusammensetzung zusätzlich eine oder mehrere der nachfolgenden Substanzen, die aus Antioxidantien, Vitaminen, Coenzymen, Fettsäuren, Aminosäuren, Co-Faktoren ein oder mehrere Süßungsmittel und/oder ein oder mehrere künstliche Süßstoffe enthält.

5. Zusammensetzung nach dem vorherigen Anspruch, wobei es sich um eine pharmazeutische, tiermedizinische, kosmetische, Nahrungsergänzungsmittel oder Nahrungsmittelzusammensetzung handelt.

6. Zusammensetzung nach einem der Ansprüche 4 oder 5, wobei das Milchsäurebakterium lebend oder tot in der Zusammensetzung vorliegt, und/oder das Milchsäurebakterium in verkapselter, sprühgetrockneter und/oder lyophilisierter Form vorliegt, und/oder das Milchsäurebakterium in einer Menge mit einem Masseanteil von 0,001 Gewichtsprozent bis 20 Gewichtsprozent, bevorzugt bis 0,005 Gewichtsprozent bis 10 Gewichtsprozent, besonders bevorzugt 0,01 Gewichtsprozent bis 5 Gewichtsprozent vorliegt.

7. Verfahren zur Identifizierung und/oder Selektion eines Milchsäurebakteriums mit der Eigenschaft, Streptococcus pyogenes zu koaggregieren, wobei das Verfahren zumindest die folgenden Schritte aufweist:
a. Inkubieren des pathogenen Mikroorganismus zur Ausbildung eines Bio filmes,
b. Zugabe eines zu untersuchenden Milchsäurebakteriums und Inkubation zu einer Mischung zur Ausbildung der Koaggregation zwischen dem pathogenen Mikroorganismus und dem zu untersuchenden Milchsäurebakteriums,
c. Abtrennen der nicht gebundenen Milchsäurebakterien durch Entfernen des Überstandes und Bestimmung des Biofilmes im Hinblick auf koaggregierte Milchsäurebakterien.

8. Verfahren nach dem vorhergehenden Anspruch, zusätzlich umfassend den Schritt:
- Untersuchung der Biofilm-Hemmung durch die pathogenen Mikroorganismen, wobei die Zugabe der zu untersuchenden Milchsäurebakterien während der Inkubation der Biofilm-bildenden pathogenen Mikroorganismen erfolgt, und/oder
zusätzlich umfassend den Schritt:
- Quantifizierung der Biofilmbildung nach Entfernung der nicht gebundenen Zellen mittels einer Messung der optischen Dichte im Vergleich zu einer Kontrolle ohne Zugabe der zu testenden Milchsäurebakterien.

9. Verwendung der Zusammensetzung nach einem oder mehreren der Ansprüche 4 bis 6 zur Herstellung eines Arzneimittels, Medizinproduktes oder Kosmetikums zur Behandlung, Prophylaxe und/oder Therapie mikrobiell bedingter Erkrankungen der Haut, der Schleimhäute und der Mundhöhle.

10. Verwendung nach einem oder mehreren der vorherigen Ansprüche, wobei die Zusammensetzung zur topischen Prophylaxe und/oder Therapie mikrobiell bedingter Erkrankungen oder entzündlicher Erkrankungen, vorzugsweise mikrobiell bedingter Erkrankungen oder entzündlicher Erkrankungen der Mundhöhle, in Form einer Kaumasse, eines Kaugummis, eines Bonbons, einer Pastille, einer Zahnpasta, eines Sprays oder einer Mundspüllösung verwendet wird/werden.

11. Verwendung der Zusammensetzung nach einem oder mehreren der Ansprüche 4 bis 6 zur Herstellung eines Reinigungsmittels oder Desinfektionsmittels für die Behandlung von Oberflächen.

12. Verwendung der Zusammensetzung nach einem oder mehreren der Ansprüche 4 bis 6 zur Herstellung eines Produktes, das im Bereich der Medizinprodukte und/oder Prophylaxe eingesetzt wird und/oder zur Herstellung eines Mittels zur oralen, sublingualen, buccalen Aufnahme und/oder zur Herstellung eines spezifisch wirkendes antimikrobielles Additiv für die lokale Behandlung von Entzündungen im Mund- und Rachenraum sowie für Infektionen des oberen Respirationstraktes und der Haut.

13. Kit zur Hygienebehandlung, umfassend Mikroorganismen nach Anspruch 1 bis 3 oder die Zusammensetzung nach den Ansprüche 4 bis 6 und körperhygienische Vorrichtungen oder Geräte, Spülungen und/oder Pasten.

## Claims

1. A lactic bacterium or its cell lysate for use in the prevention and/or treatment of microbial diseases of the skin, mucous membranes and/or oral cavity, wherein the lactic acid bacterium coaggregates with a Streptococcus pyogenes, the lactic acid bacterium being selected from the group comprising the following microorganisms deposited in the German Collection of Microorganisms and Cell Cultures and numbered as DSM 25972, DSM 25987, DSM 25988, DSM 25989, and DSM 25973.

2. The lactic acid bacterium or its cell lysate according to Claim 1, wherein coaggregation with the Streptococcus pyogenes is possible even after a biological, chemical, or physical treatment, and/or coaggregation with the Streptococcus pyogenes is possible at a pH of approximately 3 to approximately 8.

3. The lactic acid bacterium or its cell lysate according to one or more of the preceding claims, wherein the lactic acid bacterium or its cell lysate is capable of inhibiting biofilm formation by the Streptococcus pyogenes, and/or wherein the lactic acid bacterium or its cell lysate is capable of preventing Streptococcus pyogenes from binding to fibronectin, and/or wherein the lactic acid bacterium or its cell lysate is does not coaggregate with any commensal microorganism of the skin or mucous membranes.

4. A composition comprising at least one lactic acid bacterium or its cell lysate according to one or more of the preceding claims,
wherein the composition preferably comprises a pharmaceutically or cosmetically acceptable vehicle or excipient and/or
wherein the composition is in a solid, liquid or viscous form, or is an aerosol and/or
wherein the composition is in the form of a paste, soft gelatin capsule, hard gelatin capsule, powder, granules, beads, pastilles, effervescent tablet, lozenge, buccal tablet, chewable tablet, sublingual tablet, solution, tincture, emulsion, juice, concentrate, syrup, spray, drinking ampoule, gel, mouthwash, toothpaste, chewing gum, tablet, coated tablet or bonbon, and/or
wherein the composition comprises probiotics, antiseptics or other antibacterial substances, and/or
wherein the composition additionally contains one or more of the following substances: antioxidants, vitamins, coenzymes, fatty acids, and amino acids, co-factors, one or more sweeteners and/or one or more artificial sweeteners.

5. The composition according to the preceding claim, wherein the composition is a pharmaceutical, veterinary, or cosmetic composition, or a dietary supplement or dietary composition.

6. The composition according to either of Claims 4 and 5, wherein the lactic acid bacterium is viable or killed in the composition, and/or the lactic acid bacterium is in encapsulated, spray-dried, and/or lyophilized form, and/or the lactic acid bacterium is present in an amount of 0.001 wt % to 20 w %, preferably an amount of 0.005 w % to 10 w %, particularly preferably an amount of 0.01 w % to 5 w %.

7. A method for identifying and/or selecting a lactic acid bacterium that has the property of coaggregating with Streptococcus pyogenes, wherein the method comprises at least the following steps:
a. incubating the pathogenic microorganism to form a biofilm,
b. adding a lactic acid bacterium to be investigated to a mixture for coaggregation between the pathogenic microorganism and the lactic acid bacterium to be investigated, and incubating the mixture,
c. separating any unbound lactic acid bacteria by removing the supernatant, and assaying the biofilm with respect to coaggregated lactic acid bacteria.

8. The method according to the preceding claim, further comprising the step of:
- investigating the biofilm inhibition by the pathogenic microorganisms, wherein the lactic acid bacterium to be investigated is added during the incubation of the biofilm-forming pathogenic microorganism, and/or
additionally comprising the step of:
- quantifying the biofilm formation after removal of the unbound cells by measuring the optical density as compared with a control group without addition of the lactic acid bacterium to be tested.

9. Use of the composition according to one or more of Claims 4 to 6 to produce a pharmaceutical product, a medicinal product or a cosmetic product for the treatment, prevention, and/or therapy of microbial diseases of the skin, mucous membranes, and oral cavity.

10. The use according to one or more of the preceding claims, wherein the composition is used for the topical prevention and/or treatment of microbial diseases or inflammatory diseases, preferably microbial diseases or inflammatory diseases of the oral cavity, the composition being used in the form of a chewable mass, a chewing gum, a bonbon, a pastille, a toothpaste, a spray or a mouthwash.

11. The use of the composition according to one or more of Claims 4 to 6 to produce a cleaning agent or disinfectant for the treatment of surfaces.

12. The use of the composition according to one or more of Claims 4 to 6 to produce a product for use in the field of medicinal products and/or preventives, and/or to produce an agent for oral, sublingual, or buccal ingestion, and/or to produce a specifically acting antimicrobial additive for the topical treatment of inflammations in the oropharyngeal space and for infections of the upper respiratory tract and of the skin.

13. A kit for hygiene treatment comprising microorganisms according to Claims 1 to 3 or the composition according to Claims 4 to 6, along with physical hygiene devices or appliances, rinses and/or pastes.

## Revendications

1. Bactérie d'acide lactique ou son lysat cellulaire pour la prophylaxie et/ou la thérapie de maladies d'origine microbienne de la peau, des muqueuses et/ou de la cavité buccale, dans laquelle la bactérie d'acide lactique est co-agrégée avec un Streptococcus pyogenes, dans lequel la bactérie d'acide lactique est choisie dans le groupe comprenant les micro-organismes numérotés suivants, enregistrés dans la Deutsche Sammlung für Mikroorganismen und Zellkulturen sous les références DSM 25972, DSM 25987, DSM 25988, DSM 25989 et DSM 25973.

2. Bactérie d'acide lactique, ou son lysat cellulaire, selon la revendication 1, dans laquelle la capacité de co-agrégation de Streptococcus pyogenes existe même après un traitement biologique, chimique ou physique et/ou dans laquelle la capacité de co-agrégation de Streptococcus pyogenes existe à un pH entre environ 3 à environ 8.

3. Bactérie d'acide lactique, ou son lysat cellulaire selon une ou plusieurs des revendications précédentes, dans laquelle la bactérie d'acide lactique, ou son lysat cellulaire possède la capacité d'inhiber une formation d'un biofilm de Streptococcus pyogenes et/ou dans laquelle la bactérie d'acide lactique ou son lysat cellulaire présente la capacité d'inhiber la formation de fibronectine par Streptococcus pyogenes et/ou dans laquelle la bactérie d'acide lactique, ou son lysat cellulaire présente la capacité de ne co-agréger aucun micro-organisme commensal de la peau ou des muqueuses.

4. Composition comprenant au moins une bactérie d'acide lactique, ou son lysat cellulaire, selon une ou plusieurs des revendications précédentes, de préférence
dans laquelle la composition comprend un support ou un excipient, acceptables d'un point de vue pharmaceutique ou cosmétique et/ou dans laquelle la composition se présente sous forme solide, fluide, visqueuse ou sous forme d'aérosol et/ou
dans laquelle la composition se présente sous forme de pâte, de capsule de gélatine souple, de capsule de gélatine dure, de poudre, de granulés, de billes, de pastille, de comprimé effervescent, de comprimé à sucer, de comprimé gingival, de comprimé à mâcher, de comprimé sublingual, de solution, de teinture, d'émulsion, de jus, de concentré, de sirop, de spray, d'ampoule buvable, de gel, de bain de bouche, de poudre dentifrice, de gomme à mâcher, de tablette, de dragée ou de bonbon et/ou
dans laquelle la composition comprend des probiotiques, des antiseptiques ou d'autres substances actives comme antibactérien et/ou
dans laquelle la composition contient en outre une ou plusieurs des substances suivantes choisies parmi les antioxydants, les vitamines, les co-enzymes, les acides gras, les cofacteurs, un ou plusieurs agents sucrants et/ou un ou plusieurs édulcorants.

5. Composition selon la revendication précédente, qui est une composition pharmaceutique, une composition de médecine vétérinaire, une composition cosmétique, un complément alimentaire ou une composition alimentaire.

6. Composition selon l'une des revendications 4 ou 5, dans laquelle la bactérie d'acide lactique se présente sous forme vivante ou morte dans la composition, et/ou la bactérie d'acide lactique se présente sous une forme encapsulée, séchée par pulvérisation et/ou lyophilisée, et/ou la bactérie d'acide lactique se présente en une quantité ayant une proportion massique de 0,001 % en poids à 20 % en poids, de préférence jusqu'à 0,005 % en poids à 10 % en poids, de manière particulièrement préférée, de 0,01 % en poids à 5 % en poids.

7. Procédé d'identification et/ou de sélection d'une bactérie d'acide lactique présentant la propriété de co-agréger Streptococcus pyogenes, dans laquelle le procédé présente au moins les étapes suivantes :
a. incubation du micro-organisme pathogène pour la formation d'un biofilm,
b. addition d'une bactérie d'acide lactique à analyser et incubation en un mélange pour former la co-agrégation entre le micro-organisme pathogène et la bactérie d'acide lactique à analyser,
c. séparation des bactéries d'acide lactique non liées par retrait du surnageant et détermination du biofilm au regard des bactéries d'acide lactique co-agrégées.

8. Procédé selon la revendication précédente, comprenant en outre l'étape suivante :
- analyse de l'inhibition du biofilm par les micro-organismes pathogènes, dans lequel l'addition des bactéries d'acide lactique à analyser s'effectue pendant l'incubation des micro-organismes formant le biofilm et/ou
comprenant en outre l'étape suivante :
- quantification de la formation de biofilm après le retrait des cellules non liées au moyen d'une mesure de la densité optique par rapport à un témoin sans addition des bactéries d'acide lactique à tester.

9. Utilisation de la composition selon une ou plusieurs des revendications 4 à 6 pour la production d'un médicament, d'un produit médical ou d'un cosmétique pour le traitement, la prophylaxie et/ou la thérapie de maladies d'origine microbienne de la peau, des muqueuses et de la cavité buccale.

10. Utilisation selon une ou plusieurs des revendications précédentes, dans laquelle la composition est utilisée pour la prophylaxie et/ou la thérapie topique de maladies d'origine microbienne ou de maladies inflammatoires, de préférence de maladies d'origine microbienne ou de maladies inflammatoires de la cavité buccale, sous la forme d'une pâte à mâcher, d'une gomme à mâcher, d'un bonbon, d'une pastille, d'un dentifrice, d'un spray ou d'un bain de bouche.

11. Utilisation de la composition selon une ou plusieurs des revendications 4 à 6 pour la production d'un agent nettoyant ou désinfectant pour le traitement de surfaces.

12. Utilisation de la composition selon une ou plusieurs des revendications 4 à 6, pour la production d'un produit qui est utilisé dans le domaine des produits médicaux et/ou de la prophylaxie et/ou pour la production d'un agent à prise orale, sublinguale, buccale et/ou pour la production d'un additif antimicrobien à action spécifique pour le traitement local d'inflammations dans la région buccale et pharyngée ainsi que des infections des voies respiratoires hautes et de la peau.

13. Kit de traitement hygiénique, comprenant des micro-organismes selon la revendication 1 à 3 ou la composition selon les revendications 4 à 6 et dispositifs ou appareils d'hygiène corporelle, lotions de rinçage et/ou pâtes.
